# EUROPEAN PATENT SPECIFICATION

(11) **EP 0 675 886 B1**
(45) Date of publication and mention of the grant of the patent: **05.07.2000**
(21) Application number: 93922821.9
(22) Date of filing: 07.10.1993
(51) Int. Cl.: C07D 401/12, A61K 31/42, A61K 31/445, C07D 409/12, C07D 413/12, C07D 417/12, C07D 487/08, C07D 261/20, C07D 263/56, C07D 275/04, C07D 277/64

(54) **AMINOMETHYLENE SUBSTITUTED NON-AROMATIC HETEROCYCLES AND USE AS SUBSTANCE P ANTAGONISTS**
AMINOMETHYLEN SUBSTITUIERTE HETEROCYCLISCHE VERBINDUNGEN UND IHRE VERWENDUNG ALSSUBSTANZ P ANTAGONISTEN
HETEROCYCLES NON AROMATIQUES SUBSTITUES PAR AMINOMETHYLENE ET LEUR UTILISATION COMME ANTAGONISTES DE SUBSTANCE P

(30) Priority: 10.12.1992 US 988653
(43) Date of publication of application: 11.10.1995
(60) Divisional application: 97202174.5
(73) Proprietor: PFIZER INC., New York, N.Y. 10017 (US)
(72) Inventor: HOWARD, Harry, R., Bristol, CT 06010 (US); O'NEILL, Brian, T., Westbrook, CT 06498 (US)
(74) Representative: Wilkinson, Stephen John
(86) International application number: US9309407
(87) International publication number: WO9413663

(56) References cited:
- EP-A- 0 499 313
- WO-A-90/05525
- WO-A-90/05729
- WO-A-91/09844
- WO-A-91/18878
- WO-A-92/01688
- WO-A-92/06079
- WO-A-92/17449
- WO-A-92/20676
- WO-A-93/00330
- WO-A-93/01170

## Description

### Background of the Invention

The present invention relates to novel aminomethylene substituted non-aromatic heterocycles, pharmaceutical compositions comprising such compounds and the use of such compounds in the treatment and prevention of inflammatory and central nervous system disorders, as well as several other disorders. The pharmaceutically active compounds of this invention are substance P receptor antagonists. This invention also relates to novel intermediates used in the synthesis of such substance P receptor antagonists.

Substance P is a naturally occurring undecapeptide belonging to the tachykinin family of peptides, the latter being named because of their prompt stimulatory action on smooth muscle tissue. More specifically, substance P is a pharmacologically active neuropeptide that is produced in mammals and possesses a characteristic amino acid sequence that is illustrated by D. F. Veber et al. in U.S. Patent No. 4,680,283.

The following references refer, collectively, to quinuclidine, piperidine, and azanorbornane derivatives and related compounds that exhibit activity as substance P receptor antagonists: United States Patent 5,162,339, which issued on November 11, 1992; United States Patent Application 724,268, filed July 1, 1991; PCT Patent Application PCT/US 91/02853, filed April 25, 1991; PCT Patent Application PCT/US 91/03369, filed May 14, 1991; PCT Patent Application PCT/US 91/05776, filed August 20, 1991; PCT Patent Application PCT/US 92/00113, filed January 17, 1992; PCT Patent Application PCT/US 92/03571, filed May 5, 1992; PCT Patent Application PCT/US 92/03317, filed April 28, 1992; PCT Patent Application PCT/US 92/04697, filed June 11, 1992; United States Patent Application 766,488, filed September 26, 1991; United States Patent Application 790,934, filed November 12, 1991; PCT Patent Application PCT/US 92/04002, filed May 19, 1992; Japanese Patent Application 065337/92, filed March 23, 1992; and United States Patent Application 932,392, filed August 19, 1992.

Various aminomethylene substituted non-aromatic heterocyclic derivatives, which act as substance P antagonists are disclosed in the following patent applications WO-A-91 09844, WO-A-92 20676, WO-A-90 05525, WO-A-90 05729, WO-A-92 06079, WO-A-92 17449 WO-A-91 18878, WO-A-93 00330 and WO-A-93 01170

### Summary of the Invention

The present invention relates to compounds of the formula 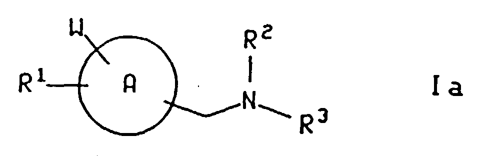
wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆)alkyl optionally substituted with from one to three fluorine atoms, -S(O)ᵥ-(C₁-C₆) alkyl wherein v is zero, one or two, halo, benzyloxy or (C₁-C₆)alkoxy optionally substituted with from one to three fluorine atoms;
R¹ is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, oxazolyl, pyridyl, pyrimidinyl, pyrazolyl or thiophenyl), wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
R² is hydrogen or -CO₂(C₁-C₁₀) alkyl;
R³ is selected from 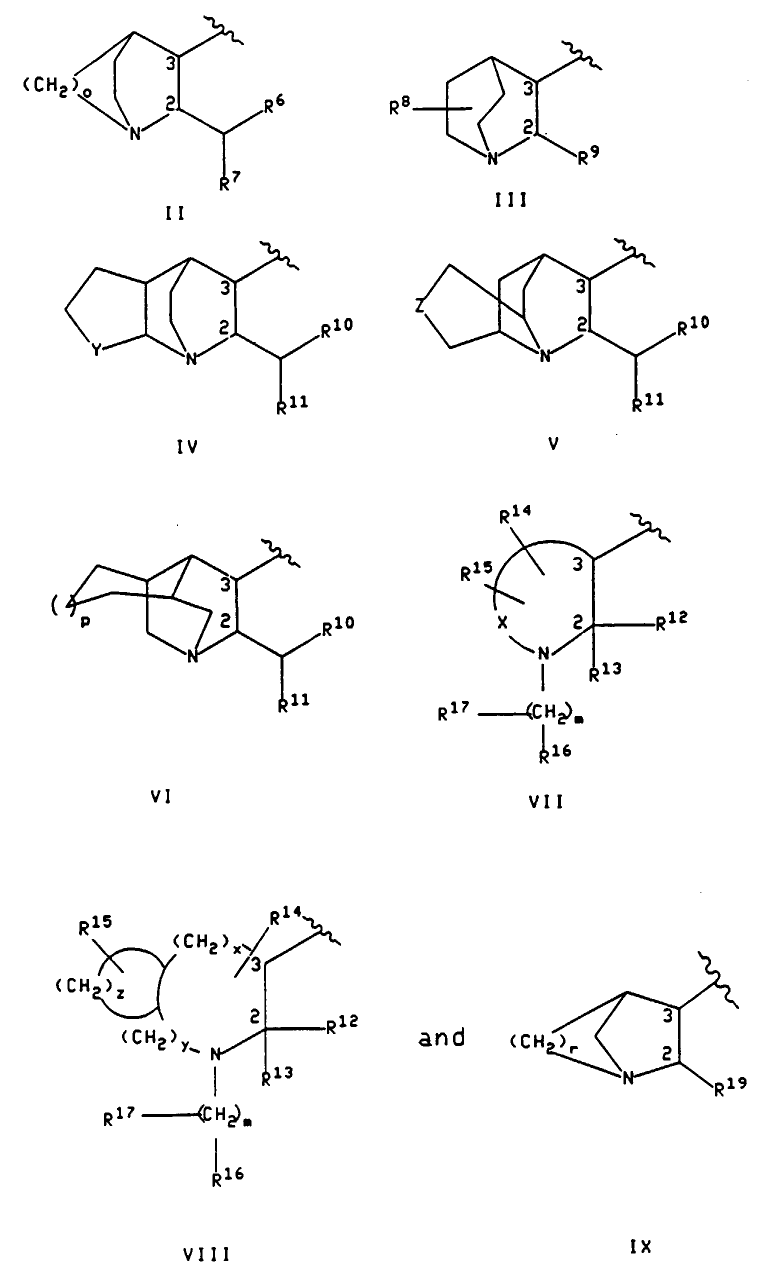
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁴ is (C₁-C₆) alkyl or phenyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 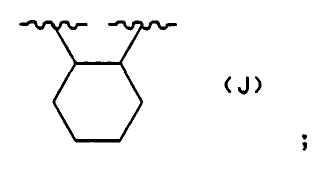
Z is oxygen, sulfur, amino, (C₁-C₃)alkylamino or (CH₂)ₙ wherein n is zero, one or two;
x is zero, one or two;
y is zero, one or two;
z is three, four or five;
o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆) alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 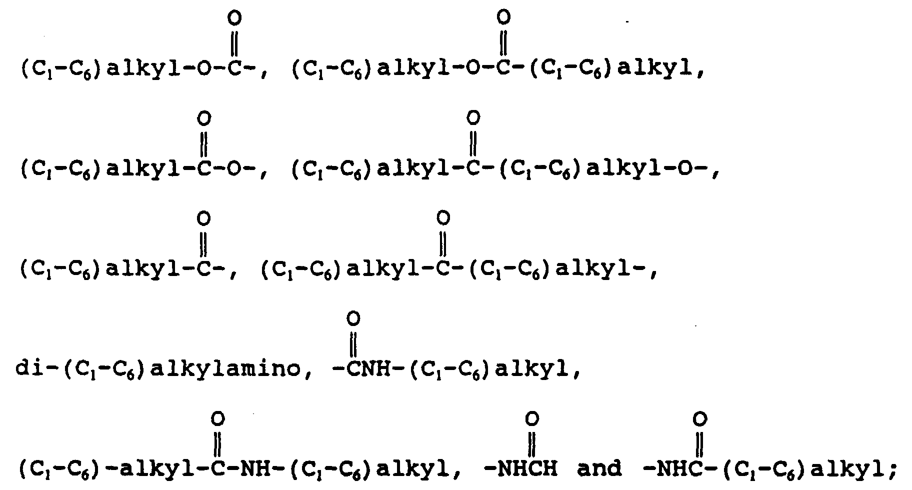 and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to such point of attachment may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 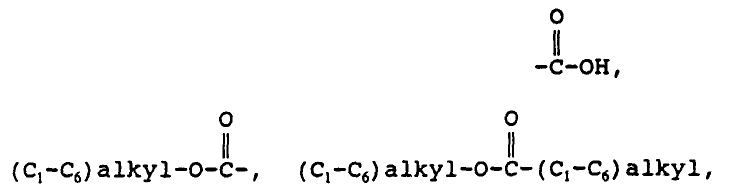 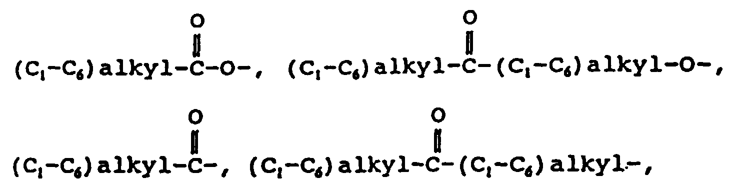 and the radicals set forth in the definition of R¹²;
R¹⁶ is 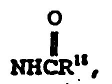 NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
with the proviso that (a) when m is 0, one of R¹⁶ and R¹⁷ is absent and the other is hydrogen, (b) when R³ is a group of the formula VIII, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ can not both be hydrogen, and (e) when R¹⁴ or R¹⁵ is attached to a carbon atom of X or (CH₂)_{y} that is adjacent to the ring nitrogen, then R¹⁴ or R¹⁵, respectively, must be a substituent wherein the point of attachment is a carbon atom.

The present invention also relates to the pharmaceutically acceptable acid addition and base salts of compounds of the formula Ia (hereinafter also referred to as compounds of the formula I). The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts. The chemical bases which are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium calcium and magnesium, etc.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined as above.

The term "one or more substituents," as used herein, includes from one to the maximum number of substituents possible based on the number of available bonding sites.

Preferred compounds of this invention include those compounds of the formula I wherein the substituents at positions "2" and "3" of the nitrogen containing ring of R³ are in a cis configuration. When R³ is a group of the formula VII or VIII, "a cis configuration", as used herein, means that the non-hydrogen substituent at position "3" is cis to R¹².

Other preferred compounds of this invention include those compounds of the formula Ia wherein R³ is a group of the formula III, VII or IX; R² is hydrogen; A is phenyl or indolinyl; W is (C₁-C₃)alkoxy optionally substituted with from one to five fluorine atoms; and R¹ is thiazolyl, imidazolyl, thiadiazolyl, pyrrolyl, oxazolyl, pyridyl, pyrimidinyl, pyrazolyl or thiophenyl, and R¹ may optionally be substituted with one or two (C₁-C₃) alkyl moieties.

More preferred compounds of this invention are the foregoing preferred compounds wherein: (a) R³ is a group of the formula III and R⁹ is benzhydryl; (b) R³ is a group of the formula VII, R¹² is phenyl, each of R¹³, R¹⁴, R¹⁵ and R¹⁶ is hydrogen, m is zero and X is -(CH₂)₃-; or (c) R³ is a group of the formula IX, r is two and R¹⁹ is benzhydryl.

Other more preferred compounds of this invention are those compounds of the formula Ia wherein: (a) R³ is a group of the formula III wherein the substituents at positions "2" and "3" of the nitrogen containing ring are in the cis configuration, R⁹ is benzhydryl and A is phenyl; or (b) R³ is a group of the formula VII wherein R¹² and the substituent at position "3" of the nitrogen containing ring are in the cis configuration, A is phenyl, R¹² is phenyl, each of R², R¹³, R¹⁴, R¹⁵ and R¹⁶ is hydrogen, m is zero, W is methoxy or isopropoxy, X is -(CH₂)₃- and R¹ is thiazolyl, imidazolyl, pyrrolyl, oxazolyl, pyridyl, pyrimidinyl, pyrazolyl, thiophenyl or thiadiazolyl.

Especially preferred compounds of the formula Ia are those wherein R³ is a group of the formula VII, each of R¹³, R¹⁴, R¹⁵ and R¹⁶ is hydrogen, m is zero, X is -(CH₂)₃-, A is phenyl, W is methoxy, and R¹ is selected from thiazolyl, imidazolyl, thiadiazolyl, pyridyl, pyrimidinyl, pyrazolyl, thiophenyl and isoxazolyl.

Specific preferred compounds of the formula I include the following:
(2S,3S)-3-[2-methoxy-5-(2-thiazolyl)benzyl]amino-2-phenylpiperidine;
(2S,3S)-3-[5-(2-imidazolyl)-2-methoxybenzyl]amino-2-phenylpiperidine;
(2S,3S)-3-[2-methoxy-5-(2-oxopyrrolidinyl)benzyl]amino-2-phenylpiperidine;
(2S,3S)-3-[2-methoxy-5-(4-methyl-2-thiazolyl)benzyl]amino-2-phenylpiperidine;
(2S,3S)-3-[2-methoxy-5-(1,2,3-thiadiazol-4-yl)benzyl]-amino-2-phenylpiperidine;
(2S,3S)-[5-(2,5-dimethyl-pyrrol-1-yl)-2-methoxybenzyl]-(2-phenylpiperidin-3-yl)amine;
(2S,3S)-3-[2-methoxy-5-(5-oxazolyl)benzyl]amino-2-phenylpiperidine;
(2S,3S)-(2-methoxy-5-pyridin-2-ylbenzyl)-(2-phenylpiperidin-3-yl)amine;
(2S,3S)-(2-methoxy-5-pyrimidin-2-ylbenzyl)-(2-phenylpiperidin-3-yl)amine;
(2S,3S)-(2-methoxy-5-pyridin-3-ylbenzyl)-(2-phenylpiperidin-3-yl)amine;
(2S,3S)-[2-methoxy-5-(6-methylpyridin-2-yl)benzyl]-(2-phenylpiperidin-3-yl)amine;
(2S,3S)-[5-(3,5-dimethylpyrazol-1-yl)-2-methoxybenzyl]-(2-phenylpiperidin-3-yl)amine;
(2S,3S)-[2-methoxy-5-(3,4,5-trimethylpyrazol-1-yl)benzyl]-(2-phenylpiperidin-3-yl)amine;
(2S,3S)-[2-isopropoxy-5-(3,4,5-trimethylpyrazol-1-yl)benzyl]-(2-phenylpiperidin-3-yl)amine;
(2S,3S)-[5-(3,5-diisopropylpyrazol-1-yl)-2-methoxybenzyl]-(2-phenylpiperidin-3-yl)amine; and
(2S,3S)-[5-(3,5-dimethylthiophen-2-yl)-2-methoxybenzyl]-(2-phenylpiperidin-3-yl)amine.

Other compounds of the formula I include the following:
(2S,3S)-5-methoxy-1-methyl-6-(2-phenylpiperidin-3-ylaminomethyl)-1,3-dihydro-indol-2-one;
(2S,3S)-(2-methoxy-5-[1,2,3]thiadiazol-4-yl-benzyl)-(2-phenyl-1-azabicyclo[2.2.2]oct-3-yl)amine;
(2S,3S)-(2-methoxy-5-[1,2,3]thiadiazol-4-yl-benzyl)-(2-benzhydryl-1-azabicyclo[2.2.2.]oct-3-yl)amine;
(2S,3S)-(2-methoxy-5-thiazol-2-yl-benzyl)-(2-benzhydryl-1-azabicyclo[2.2.2]oct-3-yl)amine;
(2S,3S)-(2-methoxy-5-[1,2,4]triazol-4-yl-benzyl)-(2-phenylpiperidin-3-yl)amine;
(2S,3S)-(2-methoxy-5-[1,2,4]triazol-1-yl-benzyl)-(2-phenylpiperidin-3-yl)amine;
(2S,3S)-(2-methoxy-5-thiazol-2-ylbenzyl)-(2-phenyldecahydroquinolin-3-yl)amine;
(2S,3S)-(2-methoxy-5-thiazol-2-ylbenzyl)-(2-phenyl-octahydro-indol-3-yl)amine; and
(2S,3S)-(2-methoxy-5-oxazol-4-ylbenzyl)-(2-phenylpiperidin-3-yl)amine.

The present invention also relates to compounds of the formulae 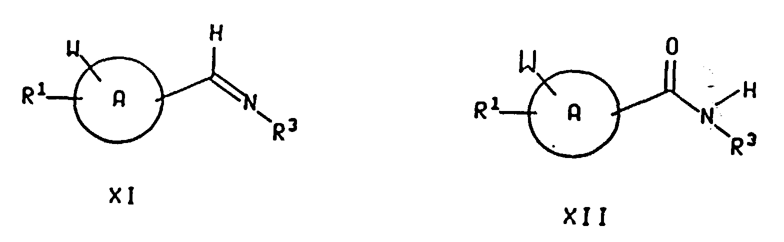 wherein ring A, R¹, R³ and W are defined as above.

Compounds of the formulae XI and XII are intermediates in the synthesis of compounds of the formula Ia.

The present invention also relates to a pharmaceutical composition for treating or preventing a condition selected from the group consisting of inflammatory diseases (e.g., arthritis, psoriasis, asthma and inflammatory bowel disease), anxiety, depression or dysthymic disorders, urinary incontinence, gastrointestinal disorders such as emesis and colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human, comprising an amount of a compound of the formula I, or a pharmaceutically acceptable salt thereof, effective in treating or preventing such condition, and a pharmaceutically acceptable carrier.

The present invention also relates to a pharmaceutical composition for antagonizing the effects of substance P in a mammal, including a human, comprising a substance P antagonizing amount of a compound of the formula I, or a pharmaceutically acceptable salt thereof, and a pharmaceutically acceptable carrier.

The present invention also relates to a pharmaceutical composition for treating or preventing a disorder in a mammal, including a human, resulting from an excess of substance P, comprising a substance P antagonizing amount of a compound of the formula I, or a pharmaceutically acceptable salt thereof, and a pharmaceutically acceptable carrier.

The present invention also relates to a pharmaceutical composition for treating or preventing a condition selected from the group consisting of inflammatory diseases (e.g., arthritis, psoriasis, asthma and inflammatory bowel disease), anxiety, depression or dysthymic disorders, urinary incontinence, gastrointestinal disorders such as emesis and colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human, comprising an amount of a compound of the formula I, or a pharmaceutically acceptable salt thereof, effective in antagonizing the effect of substance P at its receptor site, and a pharmaceutically acceptable carrier.

The present invention also relates to a pharmaceutical composition for treating or preventing a disorder in a mammal, including a human, the treatment or prevention of which is effected or facilitated by a decrease in substance P mediated neurotransmission, comprising an amount of a compound of the formula I, or a pharmaceutically acceptable salt thereof, effective in antagonizing the effect of substance P at its receptor site, and a pharmaceutically acceptable carrier.

The present invention also relates to a pharmaceutical composition for treating or preventing a disorder in a mammal, including a human, the treatment or prevention of which is effected or facilitated by a decrease in substance P mediated neurotransmission, comprising an amount of a compound of the formula I, or a pharmaceutically acceptable salt thereof, effective in treating or preventing such disorder, and a pharmaceutically acceptable carrier. neurotransmission, comprising administering to said mammal an amount of a compound of the formula I, or a pharmaceutically acceptable salt thereof, effective in treating or preventing such disorder.

The compounds of the formula I have chiral centers and therefore exist in different enantiomeric forms. This invention relates to all optical isomers and all stereoisomers of compounds of the formula I, and mixtures thereof.

### Detailed Description of the Invention

The compounds of the formula I may be prepared as described in the following reaction schemes and discussion. Unless otherwise indicated, ring A, W, R¹, R², R³, R⁴, R⁶, R⁷, R⁸, R⁹, R¹⁰, R¹¹, R¹², R¹³, R¹⁴, R¹⁵, R¹⁶, R¹⁷, R¹⁸, R¹⁹, R²², X, Z, Y, m, n, o, p, q, r, x, y, and z, and structural formulas Ia, II, III, IV, V, VI, VII, VIII, IX, XI, and XII in the reaction schemes and discussion that follow are defined as above.

As indicated above, compounds of the formula Ia are also referred to as "compounds of the formula I".

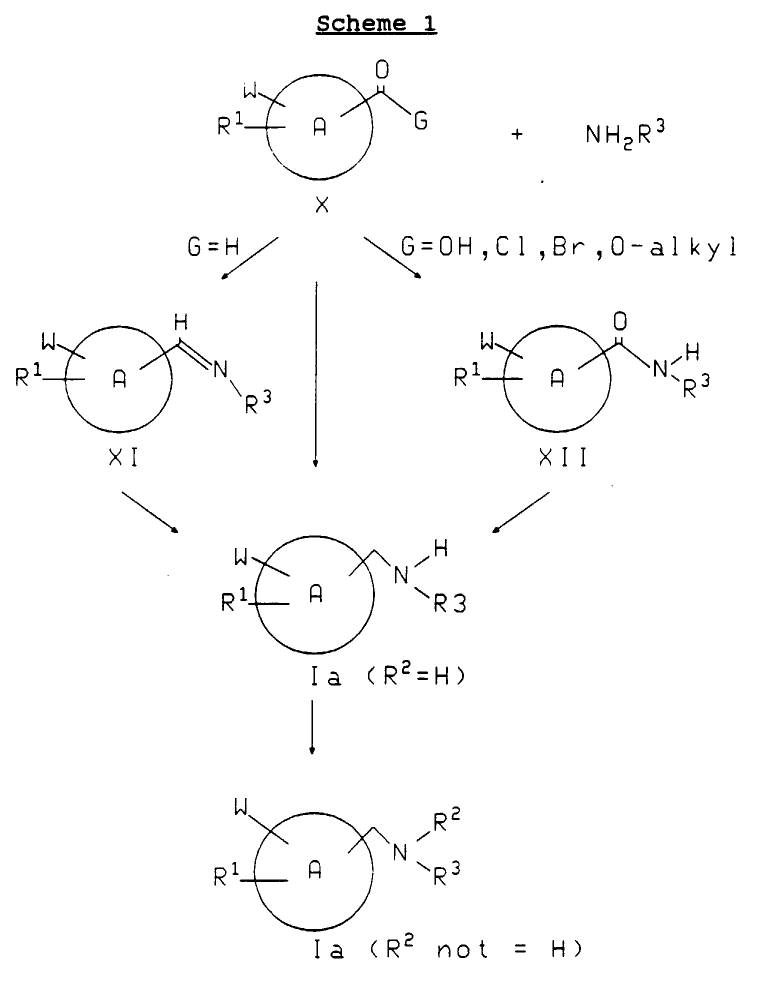

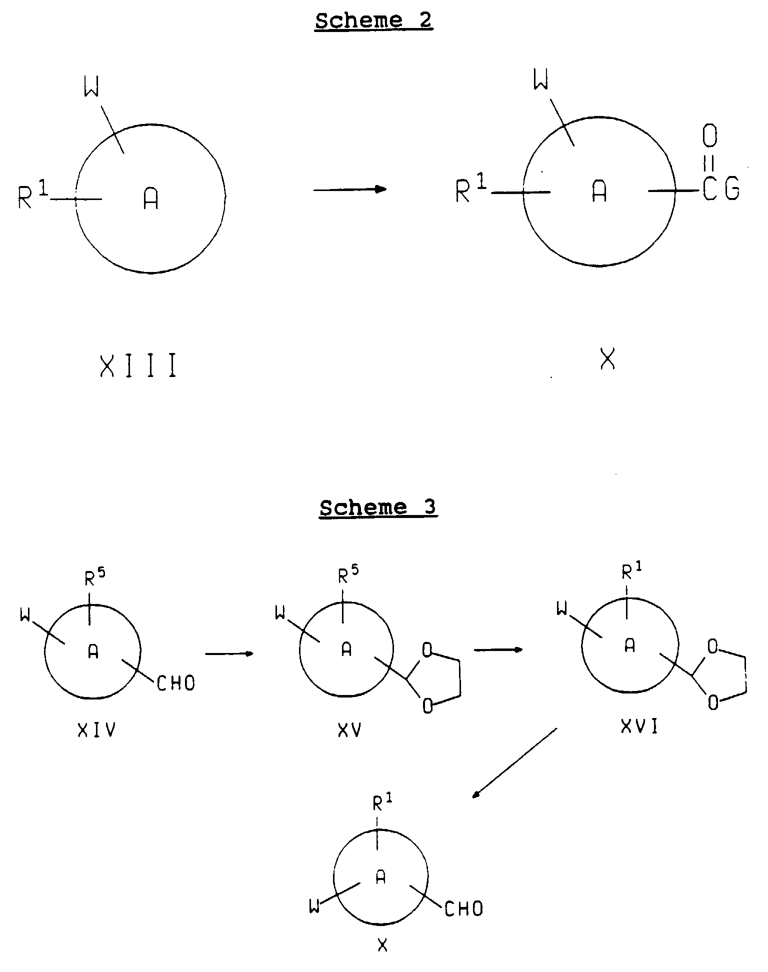

Scheme 1 illustrates the preparation of compounds of the formula Ia from starting materials of the formula X wherein G is hydrogen, hydroxy, chloro, bromo or (C₁-C₆)alkoxy.

Referring to scheme 1, a compound of the formula X wherein G is hydrogen may be converted directly into the corresponding compound of the formula I by reacting it with a compound of the formula NH₂R³ in the presence of a reducing agent. Reducing agents that may be used include sodium cyanoborohydride, sodium triacetoxyborohydride, sodium borohydride, hydrogen and a metal catalyst, zinc and hydrochloric acid, and formic acid. This reaction is typically conducted in a reaction inert solvent at a temperature from about 0°C to about 150°C. Suitable reaction inert solvents include lower alcohols (e.g., methanol, ethanol and isopropanol), 1,2-dichloroethane, acetic acid and tetrahydrofuran (THF). Preferably, the solvent is acetic acid, the temperature is about 25°C, the reducing agent is sodium triacetoxyborohydride, and the reaction is conducted in the presence of a dehydrating agent such as molecular sieves.

Alternatively, the reaction of a compound of the formula X with a compound of the formula NH₂R³ may be carried out in the presence of a dehydrating agent or by using an apparatus designed to remove azeotropically the water generated, to produce an imine of the formula 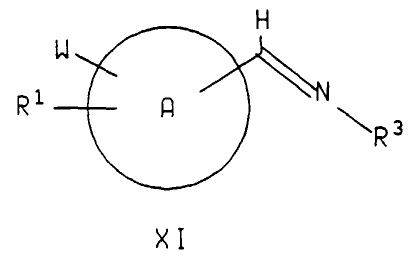 which is then reacted with a reducing agent as described above, preferably with sodium triacetoxyborohydride in an acetic acid or 1,2-dichloroethane solvent at about room temperature. The preparation of the imine XI is generally carried out in a reaction inert solvent such as benzene, xylene or toluene, preferably toluene, at a temperature from about 25°C to about 110°C, preferably at about the reflux temperature of the solvent. Suitable dehydrating agents/solvent systems include titanium tetrachloride/dichloromethane, titanium isopropoxide/dichloromethane and molecular sieves. Titanium tetrachloride/dichloromethane is preferred.

Compounds of the formula X wherein G is hydroxy, chloro, bromo or (C₁-C₆)alkoxy may be converted into the corresponding compounds of formula XII having the desired R³ group by reacting them with the appropriate compound of the formula NH₂R³ under conditions that will be obvious to those skilled in the art, and then reducing the resulting amides to yield the desired compounds having formula I wherein R² is hydrogen. When G is hydroxy, the compound of formula X is reacted with NH₂R³ in the presence of an activating agent. Appropriate activating agents include carbonyldiimidazole, chloroformates such as isobutyl chloroformate, diethylphosphoryl cyanide and dicyclohexylcarbodiimide. Carbonyldiimidazole is preferred. This reaction is generally conducted at a temperature from about 0°C to about 50°C, preferably at about 25°C, in an inert solvent such as chloroform, diethyl ether, THF or dimethylformamide (DMF).

When G is chloro or bromo, the reaction of the compound of formula X with the appropriate compound of formula NH₂R³ is typically carried out in the presence of an acid scavenger in an aprotic solvent at a temperature from about 0°C to about 100°C. Suitable acid scavengers include triethylamine (TEA), pyridine and inorganic salts such as sodium and potassium carbonate. Suitable solvents include methylene chloride (CH₂Cl₂), chloroform (CHCl₃), benzene, toluene and tetrahydrofuran (THF). Preferably, the reaction is conducted in CH₂Cl₂ at room temperature using TEA as the acid scavenger.

When G is O-(C₁-C₆)alkyl, the reaction of the compound of formula NH₂R³ is usually conducted in an aprotic solvent such as benzene, toluene, chlorobenzene or xylenes, at a temperature from about 25°C to about 100°C, preferably at about the reflux temperature of the solvent.

Reduction of the compound of formula XII so formed yields the corresponding compound of the formula I wherein R² is hydrogen. This is generally accomplished using a reducing agent such as lithium, aluminum hydride, borane dimethylsulfide complex, borane-THF or diborane, in an aprotic solvent such as THF, dioxane or diethyl ether, at a temperature from about 0°C to about 70°C. Preferably, the reducing agent is borane dimethylsulfide complex and the reaction is carried out at about room temperature in an ethereal solvent such as THF.

Compounds of the formula I wherein R² is hydrogen may be converted into the corresponding compounds wherein R² is -CO₂(C₁-C₁₀)alkyl by reacting them with a (C₁-C₁₀)alkyl halo-carbonate such as methyl or ethyl chloroformate in the presence of an acid scavenger. Typically, this reaction is conducted in an polar solvent such as chloroform, methylene chloride, water or a water/acetone mixture, at a temperature from about 0°C to about 100°C, preferably at about room temperature. Suitable acid scavengers include triethylamine, pyridine and potassium and sodium carbonate or bicarbonate.

When R³ is a group of the formula II, the starting materials of the formula NH₂R³ may be prepared as described in United States Patent 5,162,339, which issued on November 11, 1992. This patent is incorporated herein in its entirety.

When R³ is a group of the formula III, the starting materials of the formula NH₂R³ may be prepared as described in United States Patent Application Serial No. 532,525, filed June 1, 1990 and PCT Patent Application PCT/US 91/02853, filed April 25, 1991. Both these applications are incorporated herein in their entirety.

When R³ is a group of the formula IV, V or VI, the starting materials of the formula NH₂R³ may be prepared as described in United States Patent Application Serial No. 557,442, filed July 23, 1990 and PCT Patent Application PCT/US 91/03369, filed May 14, 1991. Both these applications are incorporated herein in their entirety.

When R³ is a group of the formula VII, the starting materials of the formula NH₂R³ may be prepared as described in United States Patent Application Serial No. 724,268, filed July 1, 1991, United States Patent Application Serial No. 800,667, filed November 27, 1991 and PCT Patent Application PCT/US 92/00065, filed January 14, 1992. These applications are incorporated herein in their entirety.

When R³ is a group of the formula VIII, the starting materials of the formula NH₂R³ may be prepared as described in PCT Patent Application PCT/US 91/05776, filed August 20, 1991, United States Patent Application Serial No. 800,667, filed November 27, 1991 and PCT Patent Application PCT/US 92/00065, filed January 14, 1992. These applications are incorporated herein in their entirety.

When R³ is a group of the formula IX, the starting materials of the formula NH₂R³ may be prepared as described in United States Patent Application Serial No. 719,884, filed June 21, 1991. This application is incorporated herein in its entirety.

Scheme 2 illustrates one method of preparing the starting materials of formula X wherein G is hydrogen. This is the preferred method of preparing compounds of the formula X wherein G is hydrogen and R¹ is thiazolyl, thiadiazolyl and oxazolyl. Once formed, these compounds can be converted into the corresponding compounds of the formula I or XI according to the procedures described above.

Referring to scheme 2, a compound of the formula XIII is reacted with titanium tetrachloride (TiCl₄) or tin tetrachloride (SnCl₄) and dichloromethyl methyl ether (CHCl₂-O-CH₃) at a temperature from about 0°C to about room temperature, preferably at about 0°C, in a methylene chloride or tetrachloroethylene solvent to yield the corresponding aldehyde of formula X wherein G is hydrogen. Alternatively, the compound of the formula XIII may be reacted with hexamethylene tetraamine and trifluoroacetic acid at a temperature from about 25°C to about 80°C, preferably at about 70°C, to yield the same product.

Scheme 3 illustrates a preferred method of preparing compounds of the formula X wherein G is hydrogen and R¹ is a nitrogen containing heterocyclic group (e.g., a pyrrolyl, triazolyl or imidazolyl group). Referring to scheme 3, the -CHO group of a benzaldehyde of the formula XIV is protected by conversion to the corresponding 1,3-dioxolane of formula XV, wherein R⁵ is a suitable leaving group such as iodine or bromine. This reaction is generally carried out by heating a mixture of the benzaldehyde and ethylene glycol in an inert solvent such as benzene or toluene, preferably in the presence of an acid catalyst such as p-toluenesulfonic acid, and preferably at the reflux temperature of the solvent to remove the water formed in the reaction.

The resulting compound of formula XV is then reacted with a heterocyclic compound of the formula R¹H to form the corresponding compound of formula XVI. Typically, the reaction is carried out in an aprotic, nonpolar solvent such as xylene or toluene, or in the absence of a solvent (e.g., as a melt of imidazole and the compound of the formula XV) at a temperature from about 100°C to about 300°C, in the presence of an inorganic metal catalyst such as copper metal or copper iodide, in a high pressure reactor at a pressure from about 1 atm to about 5 atm. Preferably, the reaction is carried out neat using a copper metal catalyst, at a temperature from about 140°C to about 160°C and at a pressure from about 2 atm to about 3 atm.

Treatment of the compound of formula XVI formed in the above reaction with a mixture of aqueous hydrochloric acid in acetone at a temperature from about 0°C to about 50°C, preferably at room temperature, will convert the dioxolane to the desired compound of formula X.

Alternatively, compounds of the formula X wherein G is hydrogen and R¹ does not contain an ionizable proton (for example, R¹ = 2-pyridyl, 2-thienyl or 2-pyrimidinyl) can be prepared by reacting a compound of the formula XV, as depicted in scheme 3 and defined above, wherein R⁵ is bromine or iodine, with magnesium metal to form a Grignard reagent of the formula 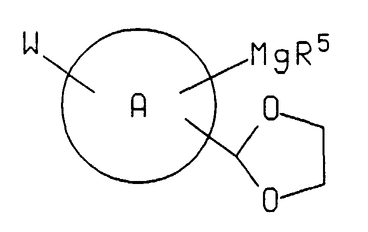 and then reacting the Grignard reagent in situ with a halogen substituted heterocyclic compound of the formula X''R¹ wherein X'' is chloro, bromo or iodo under standard Grignard conditions. These reactions are typically conducted in an ethereal solvent (e.g., diethyl ether or tetrahydrofuran), at a temperature from about 0°C to about 70°C. They are preferably conducted at the reflux temperature of the solvent in the presence of a catalyst (e.g., tetrakis (triphenylphosphine) palladium (0)).

Compounds of the formula X wherein G is other than hydrogen can be prepared from commercially available sources by methods well known to those skilled in the art. For example, compounds of the formula X wherein G is hydroxy can be obtained by: (1) oxidizing the corresponding compounds of the formula 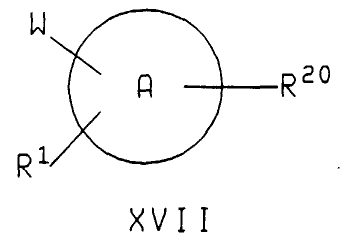 wherein R²⁰ is methyl with potassium permanganate in a reaction inert solvent such as acetone; (2) oxidizing an alcohol of the formula XVII wherein R²⁰ is hydroxymethyl with manganese dioxide; or (3) subjecting a compound of the formula XVII wherein R²⁰ is chloro, bromo or iodo to Grignard reaction conditions (i.e., reacting the compound of formula XVII with magnesium metal to form an intermediate of the formula 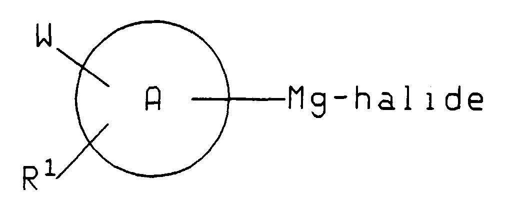 and then treating the intermediate with carbon dioxide.

The foregoing carboxylic acids of the formula X wherein G is hydroxy can be converted into the corresponding compounds of the formula X wherein G is chlorine or bromine by reaction with such reagents as sulfonyl chloride, phosphorus trichloride, phosphorus pentachloride and phosphorous tribromide.

Carboxylic esters of the formula X wherein G is (C₁-C₆)alkoxy can be prepared by a variety of methods known in the art. One such method involves reacting the corresponding acid halide in a (C₁-C₆)alkanol in the presence of a catalytic amount of hydrochloric, sulfuric or para-toluenesulfonic acid at a temperature from about room temperature to about the boiling point of the alcohol employed.

Compounds of the formula Ia wherein R¹ is pyrrolyl can also be prepared from the corresponding compounds wherein R¹ is replaced by an amino group. The corresponding amine may be obtained by reducing the corresponding nitro compound using one of several methods known to those skilled in the art. One such method involves catalytic hydrogenation of the nitro compound using hydrogen gas and a palladium on carbon catalyst in an inert solvent such as methanol or ethanol at about room temperature and a pressure of about 1-5 atm. The reduction can also be accomplished using a reducing agent such as borane/methyl sulfide in tetrahydrofuran at a temperature from about 25°C to about 70°C, preferably at the reflux temperature of the solvent. The latter reduction method is exemplified in Example 45 of United States Patent Application Serial No. 932,392, filed on August 19, 1992. This application is incorporated herein by reference in its entirety.

The amine can then be converted into the desired compound of formula I by the procedure described in Example 9.

The preparation of other compounds of the formula I not specifically described in the foregoing experimental section can be accomplished using combinations of the reactions described above that will be apparent to those skilled in the art.

In each of the reactions discussed or illustrated in schemes 1 to 3 above, pressure is not critical unless otherwise indicated. Pressures from about 0.5 atmospheres to about 5 atmospheres are generally acceptable, and ambient pressure, i.e. about 1 atmosphere, is preferred as a matter of convenience.

The novel compounds of the formula I and the pharmaceutically acceptable salts thereof are useful as substance P antagonists, i.e., they possess the ability to antagonize the effects of substance P at its receptor site in mammals, and therefore they are able to function as therapeutic agents in the treatment of the aforementioned disorders and diseases in an afflicted mammal.

The compounds of the formula I which are basic in nature are capable of forming a wide variety of different salts with various inorganic and organic acids. Although such salts must be pharmaceutically acceptable for administration to animals, it is often desirable in practice to initially isolate a compound of the Formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

Those compounds of the formula I which are also acidic in nature, e.g., where R⁶ or R¹⁰ is carboxyphenyl, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compounds of formula I and their pharmaceutically acceptable salts exhibit substance P receptor-binding activity and therefore are of value in the treatment and prevention of a wide variety of clinical conditions the treatment or prevention of which are effected or facilitated by a decrease in substance P mediated neurotransmission. Such conditions include inflammatory diseases (e.g., arthritis, psoriasis, asthma and inflammatory bowel disease), anxiety, depression or dysthymic disorders, urinary incontinence, gastrointestinal disorders such as emesis and colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis. Hence, these compounds are readily adapted to therapeutic use as substance P antagonists for the control and/or treatment of any of the aforesaid clinical conditions in mammals, including humans.

The compounds of the formula I and the pharmaceutically acceptable salts thereof can be administered via either the oral, parenteral or topical routes. In general, these compounds are most desirably administered in dosages ranging from about 5.0 mg up to about 1500 mg per day, although variations will necessarily occur depending upon the weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The compounds of the formula I and their pharmaceutically acceptable salts ("the therapeutic compounds") may be administered alone or in combination with pharmaceutically acceptable carriers or diluents by either of the three routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic compound of the present invention in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

Additionally, it is also possible to administer the therapeutic compounds of the present invention topically when treating inflammatory conditions of the skin and this may preferably be done by way of creams, jellies, gels, pastes, ointments and the like, in accordance with standard pharmaceutical practice.

The activity of the therapeutic compounds of the present invention as substance P receptor antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty- minute period. The pellet is then resuspended in 40 volumes of ice-cold 50 mM Tris buffer (pH 7.7) containing 2 mM of calcium chloride, 2 mM of magnesium chloride, 4 µg/ml of bacitracin, 4µg/ml of leupeptin, 2µg of chymostatin and 200 g/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously, been presoaked for a period of two hours prior to the filtering procedure. Radioactivity is then determined in a Beta counter at 53% counting efficiency, and the IC₅₀ values are calculated by using standard statistical methods.

The ability of the therapeutic compounds of this invention to inhibit substance P induced effects in vivo may be determined by the following procedures "a" through "d". (Procedures "a" through "c" are described in Nagahisa et al., European Journal of Pharmacology, 217, 191-5 (1992), which is incorporated herein by reference in its entirety.)

### a. Plasma extravasation in the skin

Plasma extravasation is induced by intradermal administration of substance P (50 µl, 0.01% BSA-saline solution) in dorsal skin of pentobarbital (25 mg/kg i.p.) anesthetized male Hartley guinea pigs weighing 450-500 g. The compound to be tested is dissolved in 0.1% methyl cellulose-water (MC) and dosed p.o. 1 hour before substance P challenge (3 pmol/site). Evans blue dye (30 mg/kg) is administered intravenously 5 minutes before challenge. After 10 minutes, the animals are sacrificed, the dorsal skin is removed, and the blue spots are punched out using a cork borer (11.5 mm oral dose (o.d.)). Tissue dye content is quantitated after overnight formamide extraction at 600 nm absorbance.

### b. Capsaicin-induced plasma extravasation

Plasma extravasation is induced by intraperitoneal injection of capsaicin (10 ml of 30 µM solution in 0.1% BSA/saline) into pentobarbital anesthetized (25 mg/kg i.p.) guinea pigs. The compound to be tested is dissolved in 0.1% MC and dosed p.o. 1 hour before capsaicin challenge. Evans blue dye (30 mg/kg) is administered i.v. 5 minutes before challenge. After 10 minutes, the animals are sacrificed, and both right and left ureters are removed. Tissue dye content is quantitated as in "a" above.

### c. Acetic acid-induced abdominal stretching

Male ddY mice (SLC, Japan), weighing 14-18 g, were fasted overnight. The compound to be tested is dissolved in 0.1% MC and dosed p.o. 0.5 hour before acetic acid (AA) injection (0.7%, 0.16 ml/10 g body weight). The animals are placed in clear beakers (1 per beaker) and the stretching response is counted 10 to 20 minutes after the AA injection (10 minute interval).

### d. Substance P-induced hyperlocomotor paradigm

The anti-psychotic activity of the therapeutic compounds of the present invention as neuroleptic agents for the control of various psychotic disorders may be determined by a study of their ability to suppress substance P-induced or substance P agonist induced hypermotility in guinea pigs. This study is carried out by first dosing the guinea pigs with a control compound or with an appropriate test compound of the present invention, then injecting the guinea pigs with substance P or a substance P agonist by intracerebral administration via canula and thereafter measuring their individual locomotor response to said stimulus.

The present invention is illustrated by the following examples. It will be understood, however, that the invention is not limited to the specific details of these examples.

### PREPARATION 1

### 2-Methoxy-5-(1,2,3-thiadiazol-4-yl)benzaldehyde

A mixture of 0.99 grams (5.15 mmol) of 4-(4-methoxyphenyl)-1,2,3-thiadiazole (Maybridge Chemical Co.) in 23 mL of anhydrous methylene chloride (CH₂Cl₂) cooled to 0°C, was treated with 2.3 mL (21.9 mmol) of titanium tetrachloride and stirred for 30 min. The red solution was treated with 0.97 mL (10.7 mmol) of α,α-dichloromethyl methyl ether and allowed to warm to room temperature overnight. The reaction mixture was then poured over 100 mL of saturated aqueous sodium bicarbonate (NaHCO₃), the pH was adjusted to 7-8 with solid NaHCO₃ and the solution was then extracted with CH₂Cl₂. The organic layer was dried with magnesium sulfate (MgSO₄) and concentrated to a yellow solid. Chromatography on silica gel (20% EtOAc: 80% Hexane) gave the pure title compound as a light yellow solid, 0.35 g (31%).

M.P. 156-157 °C.

¹H NMR (DMSO-d₆) δ 4.0 (s, 3H), 7.4 (d, 1H), 8.4 (m, 2H), 9.7 (s, 1H), 10.4 (s, 1H).

In the same manner, the following aldehyde intermediates were prepared:

### 2-Methoxy-5-(2-thiazolyl)benzaldehyde, 36%

M.P. 119-120°C.

¹H NMR (CDCl₃) δ 4.0 (s, 3H), 7.1 (d, 1H), 7.3 (d, 1H), 7.8 (d, 1H), 8.2 (dd, 1H), 8.3 (d, 1H), 10.5 (s, 1H).

### 2-Methoxy-5-(4-methyl-2-thiazolyl)benzaldehyde, 35%, oil

¹H NMR (CDCl₃) δ 2.46 (s, 3H), 3.95 (s, 3H), 6.82 (s, 1H), 7.02 (d, J=8.7 Hz, 1H), 8.15 (dd, 1H), 8.26 (d, J=2.4 Hz, 1H), 10.46 (s, 1H).

### 2-Methoxy-5-(5-oxazolyl)benzaldehyde, 22%

¹H NMR (CDCl₃) δ 4.0 (s, 3H), 7.1 (d, 1H), 7.4 (s, 1H), 7.8 (dd, 1H), 7.9 (s, 1H), 8.1 (d, 1H), 10.5 (s, 1H).

### 2-Methoxy-5-(6-methylpyridin-2-yl)benzoaldehyde

¹H NMR (CDCl₃, free base) δ 2.6 (s, 3H), 3.95 (s, 3H), 7.0 (m, 2H), 7.4 (d, 1H), 7.6 (d, 1H), 8.3 (dd, 1H), 8.5 (d, 1H), 10.5 (s, 1H).

Mass Spectrum (m/e, %): 228 (M⁺¹), 227 (M+, 40), 85 (100).

### 2-Methoxy-5-(pyridin-2-yl)benzaldehyde

¹H NMR (CDCl₃, free base) δ 4.0 (s, 3H), 7.1 (d, 1H), 7.2 (q, 1H), 7.7 (d, 2H), 8.3 (dd, 1H), 8.4 (d, 1H), 8.7 (d, 1H), 10.5 (s, 1H).

### 2-Methoxy-5-(pyridin-3-yl)benzaldehyde

M.p. 77-79°C.

¹H NMR (CDCl₃, free base) δ 4.0 (s, 3H), 7.2 (d, 1H), 7.4 (m, 1H), 7.8 (dd, 1H), 7.9 (dd, 1H), 8.1 (d, 1H), 8.6 (dd, 1H), 8.9 (d, 1H), 10.5 (s, 1H).

### 2-Methoxy-5-(pyrimidin-2-yl)benzaldehyde

¹H NMR (CDCl₃, free base) δ 4.0 (s, 3H), 7.1 (d, 1H), 7.2 (t, 1H), 8.7 (d, 1H), 8.8 (d, 2H), 9.0 (d, 1H), 10.5 (s, 1H).

Mass Spectrum (m/e, %): 215 (M⁺¹, 100), 214 (M+, 35).

### 2-Methoxy-5-(3,5-dimethylpyrazol-1-yl)benzaldehyde

¹H NMR (CDCl₃, free base) δ 2.4 (s, 6H), 4.0 (s 3H), 6.0 (s, 1H), 7.05 (d, 1H), 7.6 (dd, 1H), 7.85 (d, 1H), 10.5 (s, 1H).

Mass Spectrum (m/e, %): 231 (M⁺¹, 100).

### 2-Methoxy-5-(3,4,5-trimethylpyrazol-1-yl)benzaldehyde

M.p. 115-117°C.

¹H NMR (CDCl₃, free base) δ 1.9 (s, 3H), 2.1 (d, 6H), 4.0 (s, 3H), 7.0 (d, 1H), 7.8 (m, 1H), 7.9 (d, 1H), 10.5 (s, 1H).

Mass Spectrum (m/e, %): 245 (M⁺¹, 100).

### 2-Isopropoxy-5-(3,4,5-trimethylpyrazol-1-yl)benzaldehyde

¹H NMR (CDCl₃, free base) δ 1.4 (d, 6H), 2.0 (s, 3H), 2.17 (s, 3H), 2.2 (s, 3H), 4.70 (m, 1H), 7.05 (d, 1H), 7.6 (dd, 1H), 7.8 (d, 1H), 10.5 (s, 1H).

### 2-Methoxy-5-(3,5-diisopropylpyrazol-1-yl)benzaldehyde

¹H NMR (CDCl₃, free base) δ 1.2 (d, 6H), 1.3 (d, 6H), 3.0 (m, 2H), 4.0 (s, 3H), 6.1 (s, 1H), 7.0 (d, 1H), 7.6 (dd, 1H), 7.9 (d, 1H), 10.5 (s, 1H).

Mass Spectrum (m/e, %): 286 (m+, 75), 271 (100).

### 5-(3,5-Dimethylthiophen-2-yl)-2-methoxybenzaldehyde

¹H NMR (CDCl₃, free base) δ 2.25 (s, 3H), 2.5 (s, 3H), 3.9 (s, 3H), 6.9 (d, 1H), 7.15 (d, 1H), 7.25 (m, 2H), 9.8 (s, 1H).

Mass Spectrum (m/e, %): 246 (M+, 100), 231 (35).

### PREPARATION 3

### 5-(2-Imidazolyl)-2-methoxybenzaldehyde

To a solution of 2.04 grams (8.72 mmol) of 4-iodoanisole in 36 mL of CH₂Cl₂, cooled to 0°C, was added dropwise 2.0 mL (18.7 mmol) of titanium tetrachloride. After stirring for 30 min., 0.93 mL (10.3 mmol) of α,α-dichloromethyl methyl ether was added and the reaction maintained at 0°C for another 2 hours. The reaction mixture was then poured with stirring into a mixture of 50 mL of CH₂Cl₂ and 50 mL of saturated aqueous sodium bicarbonate (NaHCO₃). After 30 min., this was filtered through diatomaceous earth, the organic layer was separated, the aqueous layer was twice extracted with CH₂Cl₂, and all of the organic layers were combined, dried with MgSO₄ and concentrated in vacuo. The crude residue was recrystallized from ethanol (EtOH) to give 5-iodo-2-methoxybenzaldehyde as pale yellow needles, 1.37 grams (60%).

¹H NMR (CDCl₃) δ 4.0 (s, 3H), 6.8 (d, 1H), 7.8 (dd, 1H), 8.1 (d, 1H), 10.4 (s, 1H).

A solution of 1.0 grams (3.82 mmol) of the above aldehyde, 0.85 mL of ethylene glycol, 20 mg of p-toluenesulfonic acid and 42 mL of toluene was refluxed under nitrogen for 18 hours, cooled and concentrated in vacuo. The residue was redissolved in 50 mL of CH₂Cl₂, washed with saturated aqueous NaHCO₃, dried over MgSO₄ and concentrated to an oil. Chromatography on silica gel (10% ethyl acetate (EtOAc): 90% hexane) gave 2-(5-iodo-2-methoxyphenyl)-1,3-dioxolane as a clear oil, 0.68 grams (58%).

¹H NMR (CDCl₃) δ 3.9 (s, 3H), 4.0-4.3 (m, 4H), 6.1 (s, 1H), 6.7 (d, 1H), 7.4 (dd, 1H), 7.9 (d, 1H).

In a Pyrex high pressure reaction tube, a solution of the preceding dioxolane (200 mg, 0.66 mmol), 140 mg (2.06 mmol) of imidazole and 90 mg (1.4 mmol) of copper powder in 1 mL of anhydrous tetrahydrofuran (THF) was stirred until homogeneous. The solvent was then evaporated under nitrogen, and the tube was sealed and heated in an oil bath at 140°C for 16 hours. After cooling, the residue was dissolved in 20 mL of THF, filtered through a pad of diatomaceous earth and concentrated in vacuo to an oil. Chromatography on silica gel (100% ethyl acetate (EtOAc)) gave 60 mg of 2-(5-(2-imidazolyl)-2-methoxyphenyl)-1,3-dioxolane as a yellow oil.

MS: m/e 246 (m+).

The preceding oil in 20 mL of acetone was treated with 10 mL of 1 N hydrochloric acid (HCl) and stirred at 25°C for 3 hours, evaporated in vacuo and extracted into CH₂Cl₂. After washing with water, the organic layer was dried over MgSO₄ and concentrated to a clear oil. Chromatography on silica gel (3% CH₃OH: 97% CH₂Cl₂) gave 5-(2-imidazolyl)-2-methoxybenzaldehyde as a clear oil, 30 mg (61%).

¹H NMR (CDCl₃) δ 4.0 (s, 3H), 7.1 (d, 1H), 7.1-7.3 (m, 2H), 7.6 (dd, 1H), 7.8 (d, 2H), 10.5 (s, 1H).

### EXAMPLE 1

### (2S,3S-3-[5-(2-Imidazolyl)-2-methoxybenzyl]amino-2-phenylpiperdine trihydrochloride dihydrate

Under a nitrogen atmosphere, a mixture of 30 mg (0.15 mmol) of 5-(2-imidazolyl)-2-methoxybenzaldehyde and 54 mg (0.15 mmol) of (+)-(2S,3S)-3-amino-2-phenylpiperidine in 5 mL of dry toluene was stirred and heated to reflux for 18 hours, using a Dean-Stark trap. The toluene in the reaction flask was removed in vacuo, the residue was dissolved in 5 mL of anhydrous 1,2-dichloroethane and stirred for 10 min. Sodium triacetoxyborohydride (92 mg, 0.43 mmol) was added and stirring was continued overnight. The solvent was removed in vacuo and the residue was treated with 5 mL of water (H₂O) and extracted with methylene chloride (CH₂Cl₂) (3 x 10 mL). The organic extracts were combined, dried over magnesium sulfate (MgSO₄) and concentrated to an oil. Chromatography on silica gel using methylene chloride (CH₂Cl₂): Methanol (CH₃OH): concentrated ammonium hydroxide (NH₄OH) in a ratio of 94:5:1 gave the pure free base as a clear oil. The oil dissolved in CH₂Cl₂ was treated with a solution of Et₂O saturated with hydrogen chloride (HCl) gas to give the title product as a pale yellow solid, 15.7 mg (21%).

M.P. 235°C (decomp.)

¹H NMR (CDCl₃, free base) δ 1.5 (m, 1H), 1.7 (m, 1H), 1.9 (m, 4H), 2.2 (m, 1H), 2.9 (m, 2H), 3.3 (m, 1H), 3.5 (d, 1H), 3.6 (s, 3H), 3.7 (d, 1H), 3.9 (d, 1H), 6.7 (d, 1H), 7.0 (d, 1H), 7.1-7.4 (m, 8H), 7.7 (s, 1H).

FAB MS: m/e 363 (m+1)

Anal. calc'd for C₂₂H₂₆N₄O·3HCl·2H₂O: C, 52.03; H, 6.55; N, 11.03. Found: C, 51.84; H, 6.36; N, 10.53.

The title compounds of Examples 2 through 15 were prepared from (+)-(2S,3S)-3-amino-2-phenylpiperidine and the appropriate aldehyde using a procedure similar to that of Example 1.

### EXAMPLE 2

### (2S,3S)-3-[2-Methoxy-5-(2-thiazolyl)benzyl]amino-2-phenylpiperidine trihydrochloride dihydrate

M.P. 207°C (decomp.)

¹H NMR (CDCl₃, free base) δ 1.5 (m, 1H), 1.7 (m, 1H), 2.0 (m, 4H), 2.8 (m, 1H), 2.8 (m, 2H), 3.3 (m, 1H), 3.4 (d, 1H), 3.5 (s, 3H), 3.8 (d, 1H), 3.9 (d, 1H), 6.7 (d, 1H), 7.3 (m, 7H), 7.6 (d, 1H), 7.8 (m, 2H).

FAB MS: m/e 380 (m+1)

Anal. calc'd for C₂₂H₂₅N₃OS·3HC1·2H₂O: C, 50.34; H, 6.14; N, 8.00. Found: C, 50.24; H, 6.05; N, 7.84.

### EXAMPLE 3

### (2S,3S)-3-[2-Methoxy-5-(1,2,3-thiadiazol-4-yl) benzyl]amino-2-phenylpiperidine trihydrochloride dihydrate

M.P. > 270°C (decomp.)

¹H NMR (CDC1₃, free base) δ 1.4 (m, 1H), 1.6 (m, 1H), 1.9 (m, 3H), 2.2 (m, 2H), 3.2 (m, 2H), 3.5 (d, 4H), 3.7 (d, 1H), 3.9 (d, 1H), 6.7 (d, 1H), 7.3 (m, 5H), 7.6 (d, 1H), 7.9 (dd, 1H), 8.4 (s, 1H).

FAB MS: m/e 381 (m+1, 100%), 353, 321.

### EXAMPLE 4

### (2S,3S)-3-[2-methoxy-5-(5-oxazolyl)benzyl]amino-2-phenylpiperidine dihydrochloride

M.P. 267°C (decomp.)

¹H NMR (CDCl₃, free base) δ 1.5 (m, 1H), 1.7 (m, 1H), 2.0 (m, 4H), 2.2 (m, 1H), 2.9 (m, 2H), 3.3 (m, 1H), 3.5 (d, 1H), 3.6 (s, 3H), 3.7 (d, 1H), 3.9 (d, 1H), 6.7 (d, 1H), 7.2 (s, 1H), 7.3 (m, 6H), 7.5 (dd, 1H), 7.9 (s, 1H).

FAB MS: m/e 364 (m+1)

Anal. calc'd for C₂₂H₂₅N₃O₂·2HCl: C, 60.55; H, 6.24; N, 9.63. Found: C, 60.48; H, 6.21; N, 9.86.

### EXAMPLE 5

### (2S,3S)-3-[2-Methoxy-5-(4-methyl-2-thiazolyl)benzyl] amino-2-phenylpiperidine trihydrochloride

M.P. 239°C (decomp.)

¹H NMR (CDCl₃, free base) δ 1.5 (m, 1H), 1.7 (m, 1H), 2.2 (m, 1H), 2.6 (s, 3H), 2.9 (m, 2H), 3.3 (m, 1H), 3.4 (d, 1H), 3.5 (s, 3H), 3.6 (d, 1H), 3.8 (d, 1H), 6.7 (d, 1H), 6.8 (s, 1H), 7.2-7.4 (m, 5H), 7.6 (d, 1H), 7.8 (dd, 1H).

FAB MS: m/e 394 (m+1).

### EXAMPLE 6

### (2S,3S)-(2-Methoxy-5-pyridin-2-ylbenzyl)-(2-phenylpiperidin-3-yl)amine trihydrochloride hydrate

M.p. 230°C.

¹H NMR (CDCl₃, free base) δ 1.35-2.2 (m, 6H), 2.8 (m, 2H), 3.3 (m, 1H), 3.5 (s, 3H), 3.6 (d, 1H), 3.8 (d, 1H), 3.9 (d, 1H), 6.7 (d, 1H), 7.0-7.3 (m, 6H), 7.5 (d, 1H), 7.6 (m, 2H), 7.8 (dd, 1H), 8.6 (M+1, 100).

Mass Spectrum (m/e, %): 374 (M+1, 100).

Anal. calc'd for C₂₄H₂₇N₃O·3HCl·1/4H₂O: C, 58.69; H, 6.21; N, 8.56. Found: C, 58.60; H, 6.18; N, 8.37.

### EXAMPLE 7

### (2S,3S)-(2-Methoxy-5-pyrimidin-2-ylbenzyl)-(2-phenylpiperidin-3-yl)amine dihydrochloride hydrate

M.p. 207°C (dec.)

¹H NMR (CDCl₃, free base) δ 1.5-2.0 (m, 5H), 2.4 (m, 1H), 2.8 (m, 1H), 2.8 (m, 2H), 3.4 (m, 1H), 3.5 (s, 3H), 3.6 (d, 1H), 3.9 (d, 1H), 4.0 (d, 1H), 5.7 (d, 1H), 7.1 (t, 1H), 7.4 (m, 5H), 8.1 (d, 1H), 8.3 (dd, 1H), 8.7 (d, 2H).

Mass Spectrum (m/e, %): 375 (M+1, 100).

Anal. calc'd for C₂₃H₂₆N₄O·2HCl·2H₂O: C, 57.14; H, 6.67; N, 11.59. Found: C, 57.23; H, 6.50; N, 11.44.

### EXAMPLE 8

### (2S,3S)-(2-Methoxy-5-pyridin-3-ylbenzyl)-(2- phenylpiperidin-3-yl)amine trihydrochloride hydrate

M.p. 241°C (dec.)

¹H NMR (CDCl₃, free base) δ 1.35-2.20 (m, 6H), 2.8 (m, 2H), 3.3 (d, 1H), 3.5 (d, 1H), 3.55 (s, 3H), 3.75 (d, 1H), 3.9 (d, 1H), 6.8 (d, 1H), 7.15-7.40 (m, 8H), 7.75 (m, 1H), 8.5 (dd, 1H), 8.72 (d, 1H).

Mass Spectrum (m/e, %): 374 (M+1, 100).

Anal. calc'd for C₂₄H₂₇N₃O·3HCl·H₂O: C, 57.55; H, 6.44; N, 8.39. Found: C, 57.88; H, 6.67; N, 8.07.

### EXAMPLE 9

### (2S,3S)-[2-Methoxy-5-(6-methylpyridin-2-yl)benzyl]-(2-phenylpiperidin-3-yl)amine dihydrochloride hydrate

M.p. 215-220°C.

¹H NMR (CDCl₃, free base) δ 1.35-2.25 (m, 6H), 2.65 (s, 3H), 2.85 (m, 2H), 3.3 (d, 1H), 3.5 (s, 3H), 3.55 (d, 1H), 3.8 (d, 1H), 3.95 (d, 1H), 6.8 (d, 1H), 7.00 (d, 1H), 7.3 (m, 5H), 7.45 (d, 1H), 7.6 (m, 2H), 7.9 (dd, 1H).

Mass Spectrum (m/e, %): 387 (M+, 5), 268, 213 (100).

Anal. calc'd for C₂₅H₂₉N₃O·2HCl·1/4H₂O: C, 65.58; H, 6.83; N, 9.04. Found: C, 64.80; H, 6.76; N, 8.95.

### EXAMPLE 10

### (2S,3S)-[5-(3,5-Dimethylpyrazol-1-yl)-2-methoxybenzyl]-(2-phenylpiperidin-3-yl)amine dihydrochloride hydrate

M.p. 255-259°C.

¹H NMR (CDCl₃, free base) δ 1.4-2.2 (m, 5H), 2.15 (d, 1H), 2.2 (s, 3H), 2.3 (s, 3H), 2.8 (m, 2H), 3.25 (d, 1H), 3.45 (d, 1H), 3.5 (s, 3H), 3.75 (d, 1H), 3.9 (d, 1H), 6.0 (s, 1H), 6.7 (d, 1H), 7.05 (d, 1H), 7.15-7.35 (m, 6H).

Mass Spectrum (m/e, %): 391 (M+1, 100), 232 (20).

Anal. calc'd for C₂₄H₃₀N₄O·2HCl·1/4H₂O: C, 61.60; H, 7.00; N, 11.97. Found: C, 61.60; H, 6.97; N, 11.72.

### EXAMPLE 11

### (2S,3S)-[2-Methoxy-5-(3,4,5-trimethylpyrazol-1-yl)benzyl]-(2-phenylpiperidin-3-yl)amine trihydrochloride

M.p. 220-225°C.

¹H NMR (CDCl₃, free base) δ 1.4 (d, 1H), 1.55 (tt, 1H), 1.95 (m, 3H), 2.05 (s, 3H), 2.15 (s+m, 4H), 2.25 (s, 3H), 2.8 (m, 2H), 3.3 (d, 1H), 3.45 (d, 1H), 3.5 (s, 3H), 3.75 (d, 1H), 3.9 (d, 1H), 6.7 (d, 1H), 7.0 (d, 1H), 7.15-7.35 (m, 6H).

Mass Spectrum (m/e, %): 406 (M+2, 100).

Anal. calc'd for C₂₅H₃₂N₄O·3HCl·1/2CH₂Cl₂: C, 55.04; H, 6.52; N, 10.07. Found: C, 54.80; H, 6.82; N, 9.74.

### EXAMPLE 12

### (2S,3S)-[2-Isopropoxy-5-(3,4,5-trimethylpyrazol-1-yl)benzyl]-(2-phenylpiperidin-3-yl)amine hydrochloride hydrate

M.p. 140-155°C.

¹H NMR (CDCl₃, free base) δ 1.05 (dd, 6H), 1.45-2.0 (m, 6H), 2.05 (s, 3H), 2.15 (s, 3H), 2.25 (s, 3H), 2.80 (t, 1H), 2.9 (d, 1H), 3.3 (d, 1H), 3.4 (d, 1H), 3.65 (d, 1H), 3.9 (d, 1H), 4.4 (m, 1H), 6.75 (d, 1H), 7.0 (d, 1H), 7.1-7.35 (m, 6H).

Mass Spectrum (m/e, %): 433 (M+1, 100).

Anal. calc'd for C₂₇H₃₆N₄O·HCl·H₂O: C, 66.58; H, 8.07; N, 11.50. Found: C, 66.54; H, 8.05; N, 11.83.

### EXAMPLE 13

### (2S,3S)-[5-(3,5-Diisopropylpyrazol-1-yl)-2-methoxybenzyl]-(2-phenylpiperidin-3-yl)amine dihydrochloride hydrate

M.p. 222°C dec.

¹H NMR (CDCl₃, free base) δ 1.15 (dd, 6H), 1.3 (d, 6H), 1.35-2.15 (m, 6H), 2.85 (m, 3H), 3.05 (m, 1H), 3.25 (d, 1H), 3.45 (d, 1H), 3.5 (s, 3H), 3.7 (d, 1H), 3.9 (d, 1H), 6.0 (s, 1H), 6.7 (d, 1H), 7.0 (d, 1H), 7.15-7.35 (m, 6H).

Mass Spectrum (m/e, %): 446 (M+, 40), 232 (100).

### EXAMPLE 14

### (2S,3S)-[5-(3,5-Dimethylthiophen-2-yl)-2-methoxybenzyl](2-phenylpiperidin-3-yl)aminedihydrochloride

M.p. 252-254°C.

¹H NMR (CDCl₃, free base) δ 1.37-2.01 (m, 6H), 2.20 (s, 3H), 2.38 (d, 3H), 2.8 (m, 2H), 3.27 (d, 2H), 3.50 (d, 1H), 3.85 (s, 3H), 3.90 (d, 1H), 6.3 (d, 1H), 6.72 (d, 1H), 7.03 (m, 2H), 7.2-7.32 (m, 5H).

Mass Spectrum (m/e, %): 408 (M+2, 30), 407 (M+1, 100).

Anal. calc'd for C₂₅H₃₀N₂OS·2HCl: C, 62.62; H, 6.73; N, 5.84. Found: C, 62.29; H, 6.44; N, 5.91.

### EXAMPLE 15

### (2S,3S)-[5-(2,5-Dimethylpyrrol-1-yl)-2-methoxybenzyl]-(2-phenylpiperidin-3-yl)amine

### A. 2-Methoxy-5-nitrobenzaldehyde

A mixture of 2.5 grams (13.5 mmol) of 2-chloro-5-nitrobenzaldehyde (Aldrich Chem. Co.) in 250 mL of CH₃OH was treated with 2.92 grams (54.1 mmol) of sodium methoxide, refluxed for 16 hours and allowed to cool to room temperature. After removal of the solvent in vacuo, the residue was suspended in water, treated with 2N HCl (adjusting to pH < 5) and extracted into methylene chloride. After drying over MgSO₄ , the solvent was removed in vacuo to give a yellow solid, 2.02 grams (83%).

¹H-NMR (CDCl₃) δ 4.1 (s, 3H), 7.2 (d, 1H), 8.5 (dd, 1H), 8.7 (d, 1H), 10.5 (s, 1H).

### B. (2S,3S)-3-(2-Methoxy-5-nitrobenzyl)amino-2-phenyl-piperidine

A mixture of 0.63 grams (3.48 mmol) of the above aldehyde and 0.60 grams (3.4 mmol) of (2S,3S)-3-amino-2-phenylpiperidine (prepared as described in U.S. Patent application 724,268, filed on July 1, 1991) in 60 mL of toluene was refluxed under nitrogen for 18 hours with a Dean-Stark apparatus to remove the water generated in the reaction. The toluene was then removed in vacuo and the oily residue was redissolved in 50 mL of methanol (CH₃OH), stirred under nitrogen for 1 hour and treated with 0.16 grams (4.23 mmol) of sodium borohydride. After stirring for 24 hours at 25°C, the solvent was evaporated in vacuo and the residue was treated with 50 mL of water, stirred for 1 hour and extracted with methylene chloride. The organic extracts were combined, dried over MgSO₄ and concentrated in vacua to a pale orange oil. Chromatography on silica gel using concentrated ammonium hydroxide (NH₄OH): CH₃OH: CH₂Cl₂ (1:5:94) gave the title intermediate as a pale orange oil, 0.99 grams (85%).

¹H NMR (CDCl₃) δ 1.4 (d, 1H), 1.6 (tt, 1H), 1.7-1.95 (m, 3H), 2.05 (d, 1H), 2.75 (t, 2H), 3.15 (d, 1H), 3.35 (d, 1H), 3.55 (s, 1H), 3.65 (d, 1H), 3.85 (d, 1H), 6.65 (d, 1H), 7.2 (m, 5H), 7.9 (d, 1H), 8.0 (dd, 1H).

Mass Spectrum (m/e, %): 342 (m+1, 100%), 312 (10), 177 (16), 158 (14).

### C. (2S,3S)-3-(5-Amino-2-methoxybenzyl)amino-2-phenylpiperidine

A solution of 0.3 grams (0.88 mmol) of the preceding compound in 10 mL of ethyl acetate (EtOAc) was treated with 0.11 grams of 10% palladium on carbon and hydrogenated in a Parr Shaker apparatus at an initial hydrogen pressure of 45 psi for a total of 4 hours. The reaction mixture was filtered through a pad of diatomaceous earth (d.e.) and the d.e. cake washed with additional EtOAc. The combined organics were concentrated in vacuo to give a pale orange oil, 0.33 grams.

¹H NMR (CDCl₃) δ 1.5 (m, 1H), 1.7 (m, 1H), 2.0 (m, 1H), 2.2 (m, 1H), 2.7-2.9 (m, 6H), 3.3 (m, 1H), 3.4 (d+s, 4H), 3.6 (d, 1H), 3.9 (d, 1H)), 6.3 (d, 1H), 6.4-6.6 (m, 2H), 7.2-7.4 (m, 5H).

### D. (2S,3S)-[5-(2,5-Dimethylpyrrol-1-yl)-2-methoxybenzyl]-2-(2-phenylpiperidin-3-yl)amine

The above amine (0.33 grams) from part C in 12 mL of toluene was treated with 0.15 mL (1.28 mmol) of acetonylacetone and refluxed with a Dean-Stark apparatus for 2 hours. After cooling to 25°C, the solvent was removed in vacuo to give a yellow oil. Chromatography on silica gel using concentrated NH₄OH: CH₃OH: CH₂Cl₂ (1:5:94) gave a clear oil, 198 mg. The oil was converted to the hydrochloride salt of the title compound by suspending the free base in CH₂Cl₂ and treating it with 257 µL of 4M HCl in dioxane (2 equivalents), stirring at 25°C for 20 min, filtering and drying the solid in vacuo to give a light yellow solid, 0.19 grams.

M.P. 217°C (decomp).

¹H NMR (CDCl₃, free base) δ 1.4 (m, 1H), 1.8 (m, 1H), 1.8 (bs, 1H), 1.9 (m, 1H), 2.0 (s, 6H), 2.1 (m, 1H), 2.8 (m, 2H), 3.3 (m, 1H), 3.4 (d, 1H), 3.6 (s, 3), 3.8 (d, 1H), 3.9 (d, 1H), 5.9 (s, 2H), 6.7 (d, 1H), 6.9 (d, 1H), 7.0 (dd, 1H), 7.3 (m, 5H).

Mass spectrum: (m/e, %) 390 (m+1), 270.

Anal. calc'd for C₂₅H₃₁N₃O·2HCl·1/3CH₂Cl₂: C, 62.00; H, 6.91; N, 8.56. Found: C, 62.07; H, 6.86; N, 8.21.

## Claims

1. A compound of the formula 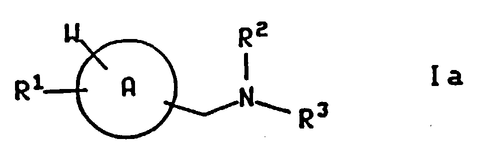
wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆) alkyl optionally substituted with from one to three fluorine atoms, -S(O)ᵥ-(C₁-C₆) alkyl wherein v is zero, one or two, halo, benzyloxy or (C₁-C₆) alkoxy optionally substituted with from one to three fluorine atoms;
R¹ is a 4, 5 or 6 membered heterocyclic ring containing from one to three heteroatoms selected from oxygen, nitrogen and sulfur, wherein said heterocyclic ring may contain from zero to three double bonds and may optionally be substituted with one or more substituents, preferably one or two substituents, independently selected from (C₁-C₆)alkyl optionally substituted with from one to three fluorine atoms and (C₁-C₆) alkoxy optionally substituted with from one to three fluorine atoms;
R² is hydrogen or -CO₂(C₁-C₁₀) alkyl;
R³ is selected from 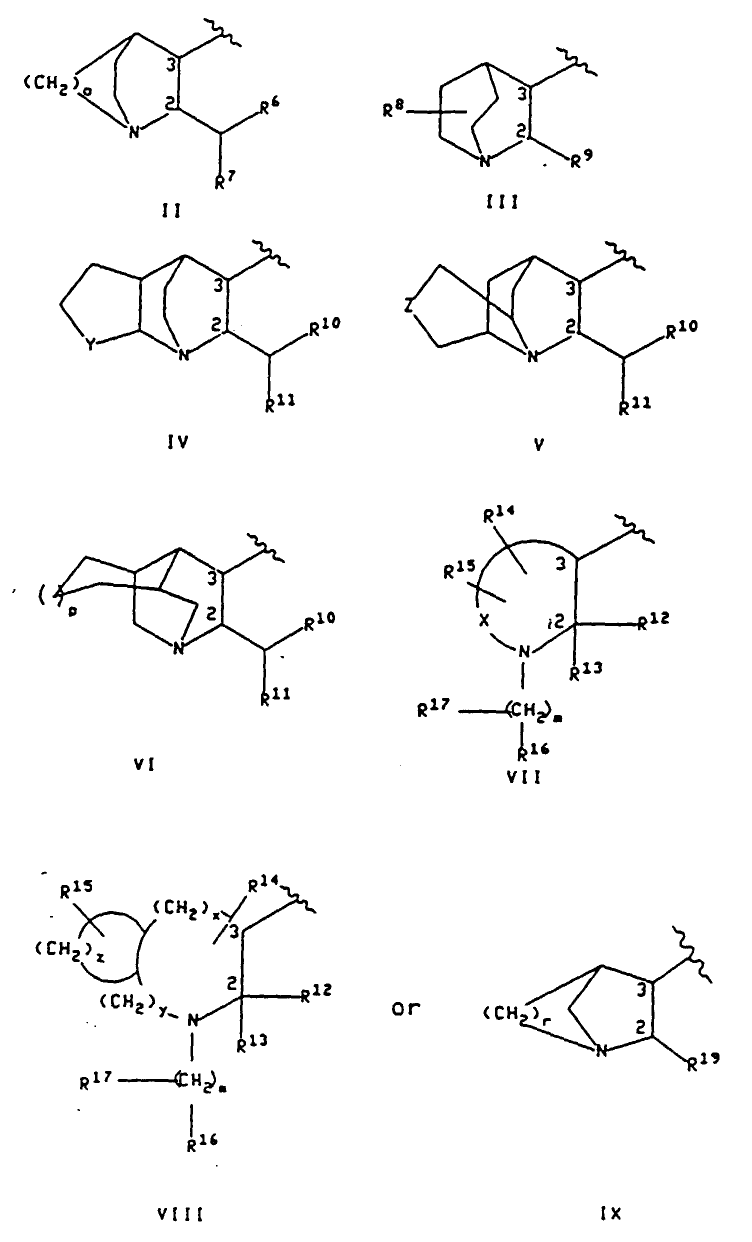
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁴ is (C₁-C₆) alkyl or phenyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 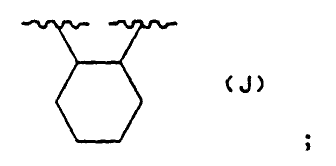
Z is oxygen, sulfur, amino, (C₁-C₃)alkylamino or (CH₂)ₙ wherein n is zero, one or two;
x is zero, one or two;
y is zero, one or two;
z is three, four or five;
o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇)cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆)alkyl and benzhydryl may optionally be substituted with one or more substituents independently selected from halo, nitro, (C₁-C₁₀)alkyl optionally substituted with from one to three fluorine atoms, (C₁-C₁₀)alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, 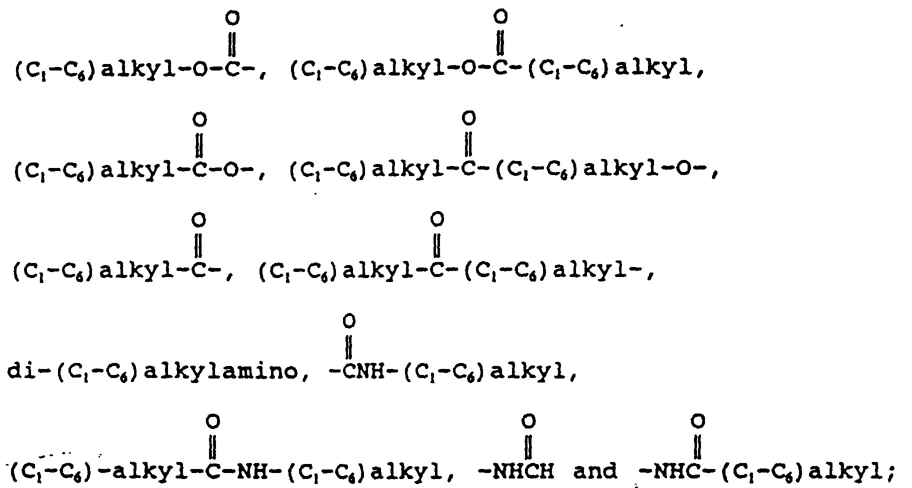 and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to it may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, 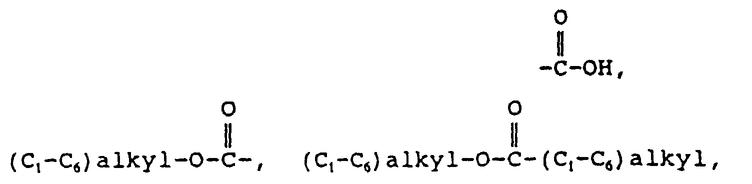 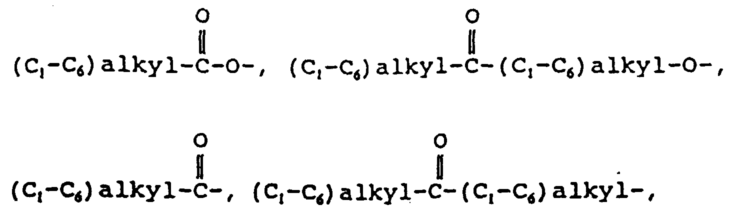 and the radicals set forth in the definition of R¹²;
R¹⁶ is NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
with the proviso that (a) when m is 0, one of R¹⁶ and R¹⁷ is absent and the other is hydrogen, (b) when R³ is a group of the formula VIII, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ cannot both be hydrogen; and (e) when R¹⁴ or R¹⁵ is attached to a carbon atom of X or (CH₂)_{y} that is adjacent to the ring nitrogen, then R¹⁴ or R¹⁵, respectively, must be a substituent wherein the point of attachment is a carbon atom;
or a pharmaceutically acceptable salt of such compound.

2. A compound according to claim 1, wherein R¹ is selected from thiazolyl, azetidinyl, pyrrolyl, pyrazolyl, 1,2,3-triazolyl, 1,2,4-triazolyl, isothiazolyl, imidazolyl, isoxazolyl, oxazolyl, pyridyl, pyrimidinyl, pyrazolyl, thiadiazolyl and thiophenyl.

3. A compound according to claim 1 or claim 2, wherein the substituents at positions "2" and "3" of the nitrogen containing ring of R³ are in a cis configuration.

4. A compound according to any one of claims 1 to 3, wherein R³ is a group of the formula III, VII or IX; R² is hydrogen; A is phenyl or indolinyl; W is (C₁-C₃)alkoxy optionally substituted with from one to five fluorine atoms; and R¹ is thiazolyl, imidazolyl, thiadiazolyl pyrrolyl, pyridyl, pyrimidinyl, pyrazolyl, thiophenyl or oxazolyl, and R¹ may optionally be substituted with one or two (C₁-C₃)alkyl moieties.

5. A compound according to any one of claims 1 to 4, wherein: (a) R³ is a group of the formula III and R⁹ is benzhydryl; (b) R³ is a group of the formula VII, R¹² is phenyl, each of R¹³, R¹⁴, R¹⁵ and R¹⁶ is hydrogen, m is zero and X is -(CH₂)₃-; or (c) R³ is a group of the formula IX, r is two and R¹⁹ is benzhydryl.

6. A compound according to claim 1 or claim 2, wherein R³ is a group of the formula III wherein the substituents at positions "2" and "3" of the nitrogen containing ring are in the cis configuration, R⁹ is benzhydryl and A is phenyl.

7. A compound according to claim 1, wherein R³ is a group of the formula VII, wherein R¹² and the substituent at position "3" of the nitrogen containing ring are in the cis configuration, A is phenyl, R¹² is phenyl, each of R², R¹³, R¹⁴, R¹⁵ and R¹⁶ is hydrogen, m is zero, W is methoxy or isopropoxy, X is -(CH₂)₃- and R¹ is thiazolyl, imidazolyl, pyrrolyl, oxazolyl, pyridyl, pyrimidinyl, pyrazolyl, thiophenyl or thiadiazolyl.

8. A compound according to claim 1 wherein R³ is a group of the formula VII, each of R¹³, R¹⁴, R¹⁵ and R¹⁶ is hydrogen, m is zero, X is -(CH₂)₃-, A is phenyl, W is methoxy and R¹ is selected from thiazolyl, imidazolyl, thiadiazolyl, pyrrolyl, pyridyl, pyrimidinyl, pyrazolyl, thiophenyl and oxazolyl.

9. A compound of the formula 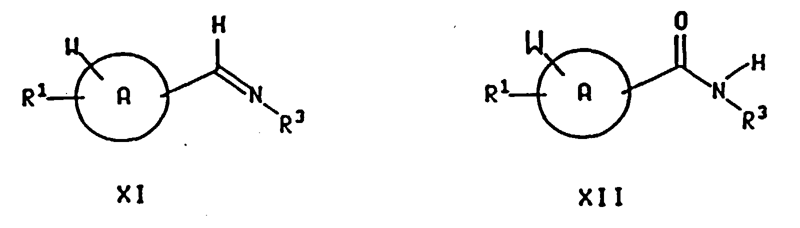 wherein W, A, R¹ and R³ are defined in claim 1.

10. A compound according to claim 1 that is selected from the group consisting of:
(2S,3S)-[5-(2,5-dimethyl-pyrrol-1-yl)-2-methoxybenzyl]-(2-phenylpiperidin-3-yl)amine;
(2S,3S)-3-[2-methoxy-5-(2-thiazolyl)benzyl]amino-2-phenylpiperidine;
(2S,3S)-3-[5-imidazolyl-2-methoxybenzyl]amino-2-phenylpiperidine;
(2S,3S)-3-[2-methoxy-5-(2-oxopyrrolidinyl)benzyl]amino-2-phenylpiperidine;
(2S,3S)-3-[2-methoxy-5-(4-methyl-2-thiazolyl)benzyl]-amino-2-phenylpiperidine;
(2S,3S)-3-[2-methoxy-5-(1,2,3-thiadiazol-4-yl)benzyl]-amino-2-phenylpiperidine;
(2S,3S)-3-[2-methoxy-5-(5-oxazolyl)benzyl]amino-2-phenylpiperidine;
(2S,3S)-(2-methoxy-5-pyridin-2-ylbenzyl)-(2-phenylpiperidin-3-yl)amine;
(2S,3S)-(2-methoxy-5-pyrimidin-2-ylbenzyl)-(2-phenylpiperidin-3-yl)amine;
(2S,3S)-(2-methoxy-5-pyridin-3-ylbenzyl)-(2-phenylpiperidin-3-yl)amine;
(2S,3S)-[2-methoxy-5-(6-methylpyridin-2-yl)benzyl]-(2-phenylpiperidin-3-yl)amine;
(2S,3S)-[5-(3,5-dimethylpyrazol-1-yl)-2-methoxybenzyl]-(2-phenylpiperidin-3-yl)amine;
(2S,3S)-[2-methoxy-5-(3,4,5-trimethylpyrazol-1-yl)benzyl]-(2-phenylpiperidin-3-yl)amine;
(2S,3S)-[2-isopropoxy-5-(3,4,5-trimethylpyrazol-1-yl)benzyl]-(2-phenylpiperidin-3-yl)amine;
(2S,3S)-[5-(3,5-diisopropylpyrazol-1-yl)-2-methoxybenzyl]-(2-phenylpiperidin-3-yl)amine; and
(2S,3S)-[5-(3,5-dimethylthiophen-2-yl)-2-methoxybenzyl]-(2-phenylpiperidin-3-yl)amine.

11. A pharmaceutical composition for treating or preventing a condition selected from the group consisting of inflammatory diseases, anxiety, urinary incontinence, gastrointestinal disorders, depression or dysthymic disorders, psychosis, pain, allergies, chronic obstructive airways disease, hypersensitivity disorders, vasospastic diseases, fibrosing and collagen diseases, reflex sympathetic dystrophy, addiction disorders, stress related somatic disorders, peripheral neuropathy, neuralgia, neuropathological disorders, disorders related to immune enhancement or suppression and rheumatic diseases in a mammal, comprising an amount of a compound according to claim 1 effective in preventing or treating such condition and a pharmaceutically acceptable carrier.

12. A pharmaceutical composition for antagonizing the effects of substance P in a mammal, comprising a substance P antagonizing effective amount of a compound according to claim 1 and a pharmaceutically acceptable carrier.

13. A pharmaceutical composition for treating or preventing a condition in a mammal, the treatment or prevention of which is effected or facilitated by a decrease in substance P mediated neurotransmission, comprising an amount of a compound according to claim 1 effective in antagonizing the effect of substance P at a receptor site and a pharmaceutically acceptable carrier.

14. A pharmaceutical composition for treating or preventing a condition in a mammal, the treatment or prevention of which is effected or facilitated by a decrease in substance P mediated neurotransmission, comprising an amount of a compound according to claim 1, or a pharmaceutically acceptable salt thereof, effective in treating or preventing such condition and a pharmaceutically acceptable carrier.

## Patentansprüche

1. Verbindung der Formel 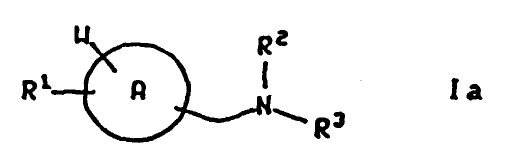
worin A ein Ringsystem darstellt, ausgewählt aus Phenyl, Naphthyl, Thienyl, Chinolinyl und Indolinyl, und worin die NR²R³ enthaltende Seitenkette an das Kohlenstoffatom von Ringsystem A gebunden ist;
W Wasserstoff, (C₁-C₆)-Alkyl, gegebenenfalls substituiert mit einem bis drei Fluoratomen, -S(O)ᵥ-(C₁-C₆)-Alkyl, worin v null, eins oder zwei ist, Halogen, Benzyloxy oder (C₁-C₆)-Alkoxy, gegebenenfalls substituiert mit einem bis drei Fluoratomen darstellt;
R¹ einen 4-, 5- oder 6-gliedrigen heterocyclischen Ring darstellt, der ein bis drei Heteroatome, ausgewählt aus Sauerstoff, Stickstoff und Schwefel, enthält, wobei der heterocyclische Ring null bis drei Doppelbindungen enthalten kann und gegebenenfalls mit einem oder mehreren Substituenten, vorzugsweise einem oder zwei Substituenten, unabhängig ausgewählt aus (C₁-C₆)-Alkyl, gegebenenfalls substituiert mit einem bis drei Fluoratomen, und (C₁-C₆)-Alkoxy, gegebenenfalls substituiert mit einem bis drei Fluoratomen, substituiert sein kann;
R² Wasserstoff oder -CO₂(C₁-C₁₀)-Alkyl darstellt,
R³ ausgewählt ist aus 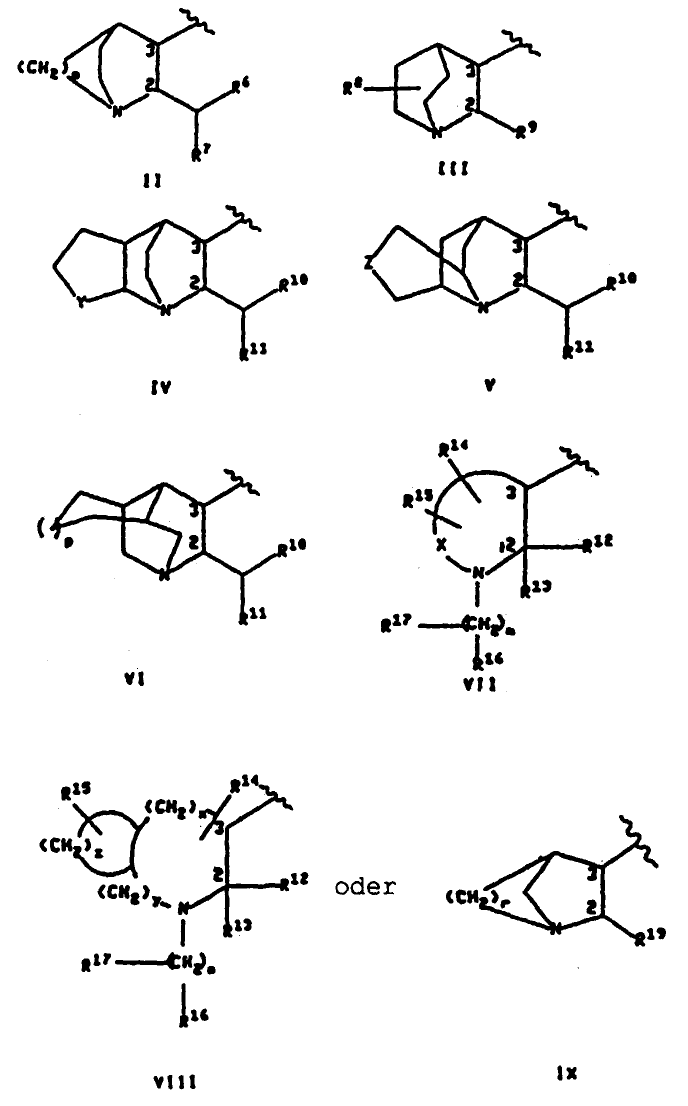
worin R⁶ und R¹⁰ unabhängig voneinander ausgewählt sind aus Furyl, Thienyl, Pyridyl, Indolyl, Biphenyl und Phenyl, wobei das Phenyl gegebenenfalls mit einem oder zwei Substituenten, unabhängig ausgewählt aus Halogen, (C₁-C₁₀)-Alkyl, gegebenenfalls substituiert mit einem bis drei Fluoratomen, (C₁-C₁₀)-Alkoxy, gegebenenfalls substituiert mit einem bis drei Fluoratomen, Carboxy, Benzyloxycarbonyl und (C₁-C₃)-Alkoxycarbonyl, substituiert sein kann;
R⁴ (C₁-C₆)-Alkyl oder Phenyl darstellt;
R⁷ aus verzweigtem (C₃-C₄)-Alkyl, verzweigtem (C₅-C₆)-Alkenyl, (C₅-C₇)-Cycloalkyl und den Resten, die in der Definition von R⁶ genannt wurden, ausgewählt ist;
R⁸ Wasserstoff oder (C₁-C₆)-Alkyl darstellt;
R⁹ und R¹⁹ unabhängig voneinander aus Phenyl, Biphenyl, Naphthyl, Pyridyl, Benzhydryl, Thienyl und Furyl ausgewählt sind, und R⁹ und R¹⁹ gegebenenfalls mit einem bis drei Substituenten, unabhängig ausgewählt aus Halogen, (C₁-C₁₀)-Alkyl, gegebenenfalls substituiert mit einem bis drei Fluoratomen, und (C₁-C₁₀)-Alkoxy, gegebenenfalls substituiert mit einem bis drei Fluoratomen, substituiert sein können;
Y (CH₂)ₗ bedeutet, worin l eine ganze Zahl von eins bis drei ist oder Y eine Gruppe der Formel 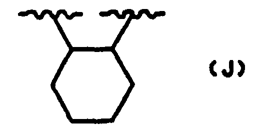 darstellt;
Z Sauerstoff, Schwefel, Amino, (C₁-C₃)-Alkylamino oder (CH₂)ₙ darstellt, worin n null, eins oder zwei ist;
x null, eins oder zwei ist,
y null, eins oder zwei ist,
z drei, vier oder fünf ist,
o zwei oder drei ist,
p null oder eins ist,
r eins, zwei oder drei ist,
wobei der (CH₂)_{z}-enthaltende Ring null bis drei Doppelbindungen enthalten kann, und eines der Kohlenstoffatome von (CH₂)_{z} gegebenenfalls durch Sauerstoff, Schwefel oder Stickstoff ersetzt sein kann;
R¹¹ Thienyl, Biphenyl oder Phenyl, gegebenenfalls substituiert mit einem oder zwei Substituenten, unabhängig ausgewählt aus Halogen, (C₁-C₁₀)-Alkyl, gegebenenfalls substituiert mit einem bis drei Fluoratomen, und (C₁-C₁₀)-Alkoxy, gegebenenfalls substituiert mit einem bis drei Fluoratomen, darstellt;
X (CH₂)_{q} bedeutet, worin q eine ganze Zahl von 1 bis 6 ist, und worin eine beliebige oder beliebige der Kohlenstoff-Kohlenstoff-Einfachbindungen in dem (CH₂)_{q} gegebenenfalls durch eine Kohlenstoff-Kohlenstoff-Doppelbindung ersetzt sein kann, und worin ein beliebiges oder beliebige der Kohlenstoffatome von dem (CH₂)_{q} gegebenenfalls mit R¹⁴ substituiert sein kann, und worin ein beliebiges oder beliebige der Kohlenstoffatome des (CH₂)_{q} gegebenenfalls mit R¹⁵ substituiert sein kann;
m eine ganze Zahl von 0 bis 8 ist und eine beliebige oder beliebige der Kohlenstoff-Kohlenstoff-Einfachbindungen von (CH₂)ₘ, worin beide Kohlenstoffatome einer solchen Bindung aneinander und an ein anderes Kohlenstoffatom in der Kette (CH₂)ₘ gebunden sind, gegebenenfalls durch eine Kohlenstoff-Kohlenstoff-Doppelbindung oder eine Kohlenstoff-Kohlenstoff-Dreifachbindung ersetzt sein können, und ein beliebiges oder beliebige der Kohlenstoffatome von dem (CH₂)ₘ gegebenenfalls mit R¹⁷ substituiert sein kann;
R¹² einen Rest, ausgewählt aus Wasserstoff, geradkettigen oder verzweigtkettigen (C₁-C₆)-Alkyl, (C₃-C₇)-Cycloalkyl, worin eines der Kohlenstoffatome gegebenenfalls durch Stickstoff, Sauerstoff oder Schwefel ersetzt sein kann; Aryl, ausgewählt aus Biphenyl, Phenyl, Indanyl und Naphthyl; Heteroaryl, ausgewählt aus Thienyl, Furyl, Pyridyl, Thiazolyl, Isothiazolyl, Oxazolyl, Isoxazolyl, Triazolyl, Tetrazoyl und Chinolyl; Phenyl-(C₂-C₆)-alkyl, Benzhydryl und Benzyl bedeutet, wobei der Punkt der Bindung an R¹² ein Kohlenstoffatom ist, sofern R¹² nicht Wasserstoff darstellt, und wobei jede der Aryl- und Heteroarylgruppen und der Phenyleinheiten des Benzyl, Phenyl-(C₂-C₆)-alkyl und Benzhydryl, gegebenenfalls mit einem oder mehreren Substituenten, unabhängig ausgewählt aus Halogen, Nitro, (C₁-C₁₀)-Alkyl, gegebenenfalls substituiert mit einem bis drei Fluoratomen, (C₁-C₁₀)-Alkoxy, gegebenenfalls substituiert mit einem bis drei Fluoratomen, Amino, Hydroxy-(C₁-C₆)-alkyl, (C₁-C₆)-Alkoxy-(C₁-C₆)-alkyl, (C₁-C₆)-Alkylamino, 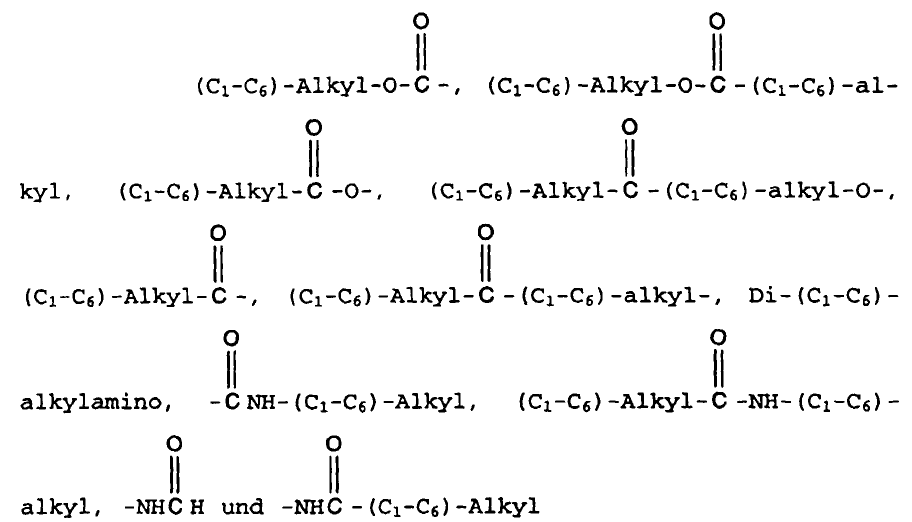 substituiert sein kann; und wobei eine der Phenyleinheiten des Benzhydryl gegebenenfalls durch Naphthyl, Thienyl, Furyl oder Pyridyl ersetzt sein kann;
R¹³ Wasserstoff, Phenyl oder (C₁-C₆)-Alkyl darstellt; oder R¹² und R¹³ zusammen mit dem Kohlenstoffatom, an das sie gebunden sind, einen gesättigten carbocyclischen Ring mit drei bis sieben Kohlenstoffatomen bilden, wobei eines der Kohlenstoffatome, das weder am Punkt der Bindung des Spirorings noch benachbart dazu ist, gegebenenfalls durch Sauerstoff, Stickstoff oder Schwefel ersetzt sein kann;
R¹⁴ und R¹⁵ jeweils unabhängig ausgewählt sind aus Wasserstoff, Hydroxy, Halogen, Amino, Oxo(=O), Cyano, Hydroxy-(C₁-C₆)-alkyl, (C₁-C₆)-Alkoxy-(C₁-C₆)-alkyl, (C₁-C₆)-Alkylamino, Di-(C₁-C₆)-alkylamino, (C₁-C₆)-Alkoxy, 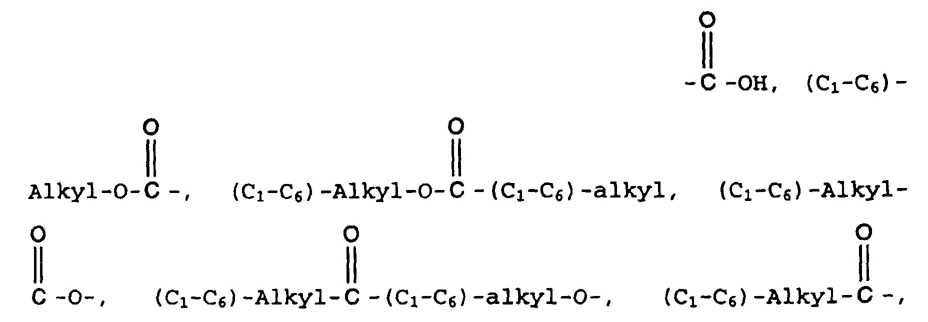 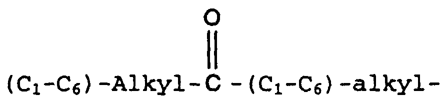 und den in der Definition von R¹² angeführten Resten;
R¹⁶ 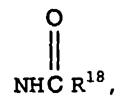 NHCH₂R¹⁸, SO₂R¹⁸, CO₂H oder einen der für eine beliebige oder beliebige der Definitionen von R¹², R¹⁴ und R¹⁵ angeführten Reste darstellt;
R¹⁷ Oximino (=NOH) oder einen der für eine beliebige oder beliebige der Definitionen von R¹², R¹⁴ und R¹⁵ angeführten Resten darstellt; und
R¹⁸ (C₁-C₆)-Alkyl, Wasserstoff, Phenyl oder Phenyl(C₁-C₆)-alkyl darstellt,
mit der Maßgabe, daß (a) wenn m 0 ist, einer der Reste R¹⁶ und R¹⁷ nicht vorliegt und der andere Wasserstoff darstellt, (b) wenn R³ eine Gruppe der Formel VIII darstellt, R¹⁴ und R¹⁵ nicht an das gleiche Kohlenstoffatom gebunden sein können, (c) wenn R¹⁴ und R¹⁵ an das gleiche Kohlenstoffatom gebunden sind, dann entweder jeder der Reste R¹⁴ und R¹⁵ unabhängig ausgewählt ist aus Wasserstoff, Fluor, (C₁-C₆)-Alkyl, Hydroxy-(C₁-C₆)-alkyl und (C₁-C₆)-Alkoxy-(C₁-C₆)-alkyl, oder R¹⁴ und R¹⁵ zusammen mit dem Kohlenstoffatom, an das sie gebunden sind, einen gesättigten carbocyclischen (C₃-C₆)-Ring bilden, der eine Spiroverbindung mit dem Stickstoff enthaltenden Ring, an den sie gebunden sind, bildet; (d) R¹² und R¹³ nicht beide Wasserstoff sein dürfen; und (e) wenn R¹⁴ oder R¹⁵ an das Kohlenstoffatom von X oder (CH₂)_{y} gebunden ist, das zu dem Ring-Stickstoffatom benachbart ist, dann R¹⁴ bzw. R¹⁵ einen Substituenten darstellen muß, bei dem der Punkt der Bindung ein Kohlenstoffatom darstellt;
oder ein pharmazeutisch verträgliches Salz einer solchen Verbindung.

2. Verbindung nach Anspruch 1, worin R¹ ausgewählt ist aus Thiazolyl, Azetidinyl, Pyrrolyl, Pyrazolyl, 1,2,3-Triazolyl, 1,2,4-Triazolyl, Isothiazolyl, Imidazolyl, Isoxazolyl, Oxazolyl, Pyridyl, Pyrimidinyl, Pyrazolyl, Thiadiazolyl und Thiophenyl.

3. Verbindung nach Anspruch 1 oder Anspruch 2, worin die Substituenten in Stellungen 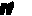 2" und 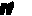 3" des Stickstoff enthaltenden Rings von R³ in einer cis-Konfiguration vorliegen.

4. Verbindung nach einem der Ansprüche 1 bis 3, worin R³ eine Gruppe der Formel III, VII oder IX darstellt; R² Wasserstoff darstellt; A Phenyl oder Indolinyl darstellt; W (C₁-C₃)-Alkoxy, gegebenenfalls substituiert mit einem bis fünf Fluoratomen darstellt; und R¹ Thiazolyl, Imidazolyl, Thiadiazolyl, Pyrrolyl, Pyridyl, Pyrimidinyl, Pyrazolyl, Thiophenyl oder Oxazolyl darstellt und R¹ gegebenenfalls mit einer oder zwei (C₁-C₃)-Alkyleinheiten substituiert sein kann.

5. Verbindung nach einem der Ansprüche 1 bis 4, worin: (a) R³ eine Gruppe der Formel III darstellt und R⁹ Benzhydryl darstellt; (b) R³ eine Gruppe der Formel VII darstellt, R¹² Phenyl darstellt, jeder der Reste R¹³, R¹⁴, R¹⁵ und R¹⁶ Wasserstoff darstellt, m null ist und X -(CH₂)₃- darstellt, oder (c) R³ eine Gruppe der Formel IX darstellt, r zwei ist und R¹⁹ Benzhydryl darstellt.

6. Verbindung nach Anspruch 1 oder Anspruch 2, worin R³ eine Gruppe der Formel III darstellt, worin die Substituenten in Stellungen 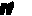 2 und 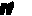 3" des Stickstoff enthaltenden Rings in der cis-Konfiguration vorliegen, R⁹ Benzhydryl ist und A Phenyl ist.

7. Verbindung nach Anspruch 1, worin R³ eine Gruppe der Formel VII darstellt, worin R¹² und der Substituent in Stellung 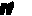 3" des Stickstoff enthaltenden Rings in der cis-Konfiguration vorliegen, A Phenyl darstellt, R¹² Phenyl darstellt, jeder der Reste R², R¹³, R¹⁴, R¹⁵ und R¹⁶ Wasserstoff darstellt, m null ist, W Methoxy oder Isopropoxy darstellt, X -(CH₂)₃- darstellt und R¹ Thiazolyl, Imidazolyl, Pyrrolyl, Oxazolyl, Pyridyl, Pyrimidinyl, Pyrazolyl, Thiophenyl oder Thiadiazolyl darstellt.

8. Verbindung nach Anspruch 1, worin R³ eine Gruppe der Formel VII darstellt, jeder der Reste R¹³, R¹⁴, R¹⁵ und R¹⁶ Wasserstoff darstellt, m null ist, X -(CH₂)₃- darstellt, A Phenyl darstellt, W Methoxy darstellt und R¹ ausgewählt ist aus Thiazolyl, Imidazolyl, Thiadiazolyl, Pyrrolyl, Pyridyl, Pyrimidinyl, Pyrazolyl, Thiophenyl und Oxazolyl.

9. Verbindung der Formel 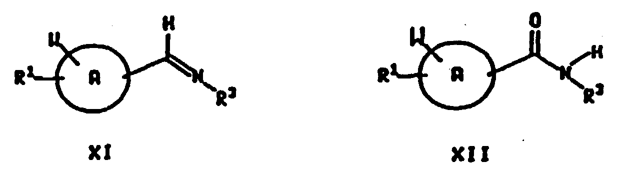 worin W, A, R¹ und R³ wie in Anspruch 1 definiert sind.

10. Verbindung nach Anspruch 1, ausgewählt aus der Gruppe bestehend aus:
(2S,3S)-[5-(2,5-Dimethlylpyrrol-1-yl)-2-methoxybenzyl]-(2-phenylpiperidin-3-yl)amin;
(2S,3S)-3-[2-Methoxy-5-(2-thiazolyl)benzyl]amino-2-phenylpiperidin;
(2S,3S)-3-[5-Imidazolyl-2-methoxybenzyl]amino-2-phenylpiperidin;
(2S,3S)-3-[2-Methoxy-5-(2-oxopyrrolidinyl)benzyl]-amino-2-phenylpiperidin;
(2S,3S)-3-[2-Methoxy-5-(4-methyl-2-thiazolyl)benzyl]amino-2-phenylpiperidin;
(2S,3S)-3-[2-Methoxy-5-(1,2,3-thiadiazol-4-yl)benzyl]amino-2-phenylpiperidin;
(2S,3S)-3-[2-Methoxy-5-(5-oxazolyl)benzyl]amino-2-phenylpiperidin;
(2S,3S)-(2-Methoxy-5-pyridin-2-yl-benzyl)-(2-phenylpiperidin-3-yl)amin;
(2S,3S)-(2-Methoxy-5-pyrimidin-2-yl-benzyl)-(2-phenylpiperidin-3-yl)amin;
(2S,3S)-(2-Methoxy-5-pyridin-3-yl-benzyl)-(2-phenylpiperidin-3-yl)amin;
(2S,3S)-[2-Methoxy-5-(6-methylpyridin-2-yl)benzyl]-(2-phenylpiperidin-3-yl)amin;
(2S,3S)-[5-(3,5-Dimethylpyrazol-1-yl)-2-methoxybenzyl]-(2-phenylpiperidin-3-yl)amin;
(2S,3S)-[2-Methoxy-5-(3,4,5-trimethylpyrazol-1-yl)-benzyl]-(2-phenylpiperidin-3-yl)amin;
(2S,3S)-[2-Isopropoxy-5-(3,4,5-trimethylpyrazol-1-yl)benzyl]-(2-phenylpiperidin-3-yl)amin;
(2S,3S)-[5-(3,5-Diisopropylpyrazol-1-yl)-2-methoxybenzyl]-(2-phenylpiperidin-3-yl)amin und
(2S,3S)-[5-(3,5-Dimethylthiophen-2-yl)-2-methoxybenzyl]-(2-phenylpiperidin-3-yl)amin.

11. Pharmazeutische Zusammensetzung zur Behandlung oder Prävention eines Zustands, ausgewählt aus der Gruppe, bestehend aus entzündlichen Erkrankungen, Beklemmung, Harninkontinenz, gastrointestinalen Erkrankungen, Depression oder disthymischen Erkrankungen, Psychose, Schmerz, Allergien, chronischer obstruktiver Luftwegserkrankung, Hypersensibilitätserkrankungen, vasospastischen Erkrankungen, fibrosierenden Erkrankungen und Collagenerkrankungen, reflexsympathetischer Dystrophie, Suchterkrankungen, stressbedingten somatischen Erkrankungen, peripherer Neuropathie, Neuralgie, neuropathologischen Erkrankungen, Erkrankungen, die mit Immunverstärkung oder -schwächung einhergehen, und rheumatischen Erkrankungen bei einem Säuger, umfassend eine Menge einer Verbindung nach Anspruch 1, die bei der Prävention oder Behandlung eines solchen Zustands wirksam ist, und einen pharmazeutisch verträglichen Träger.

12. Pharmazeutische Zusammensetzung zum Entgegenwirken der Wirkungen von Substanz P in einem Säuger, umfassend eine wirksame Substanz P-entgegenwirkende Menge einer Verbindung nach Anspruch 1 und einen pharmazeutisch verträglichen Träger.

13. Pharmazeutische Zusammensetzung zur Behandlung oder Prävention eines Zustands bei einem Säuger, wobei die Behandlung oder Prävention davon durch eine Senkung der Substanz P-vermittelten Neurotransmission bewirkt oder erleichtert wird, umfassend eine Menge einer Verbindung nach Anspruch 1, die beim Entgegenwirken der Wirkung von Substanz P an einer Rezeptorstelle wirksam ist, und einen pharmazeutisch verträglichen Träger.

14. Pharmazeutische Zusammensetzung zur Behandlung oder Prävention eines Zustands bei einem Säuger, wobei die Behandlung oder Prävention davon durch eine Senkung der Substanz P-vermittelten Neurotransmission bewirkt oder erleichtert wird, umfassend eine Menge einer Verbindung nach Anspruch 1 oder eines pharmazeutisch verträglichen Salzes davon, die bei der Behandlung oder Prävention eines solchen Zustands wirksam ist, und einen pharmazeutisch verträglichen Träger.

## Revendications

1. Composé de formule 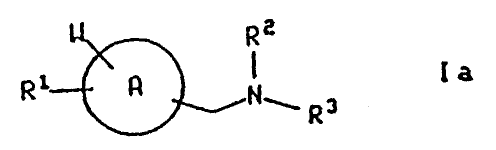
dans laquelle A est un système cyclique choisi entre phényle, naphtyle, thiényle, quinolinyle et indolinyle et dans laquelle la chaîne latérale contenant le groupe NR²R³ est liée à un atome de carbone du système cyclique A ;
W est l'hydrogène, un groupe alkyle en C₁ à C₆ facultativement substitué avec un à trois atomes de fluor, un groupe -S(O)ᵥ-(alkyle en C₁ à C₆) dans lequel v a la valeur zéro, un ou deux, un groupe halogéno, benzyloxy ou alkoxy en C₁ à C₆ facultativement substitué avec un à trois atomes de fluor ;
R¹ est un noyau hétérocyclique de 4, 5 ou 6 atomes contenant un à trois hétéroatomes choisis entre oxygène, azote et soufre, ce noyau hétérocyclique pouvant contenir zéro à trois doubles liaisons et pouvant facultativement être substitué avec un ou plusieurs substituants, de préférence un ou deux substituants, choisis indépendamment entre alkyle en C₁ à C₆ facultativement substitué avec un à trois atomes de fluor et alkoxy en C₁ à C₆ facultativement substitué avec un à trois atomes de fluor ;
R² est l'hydrogène ou un groupe -CO₂(alkyle en C₁ à C₁₀) ;
R³ est choisi entre 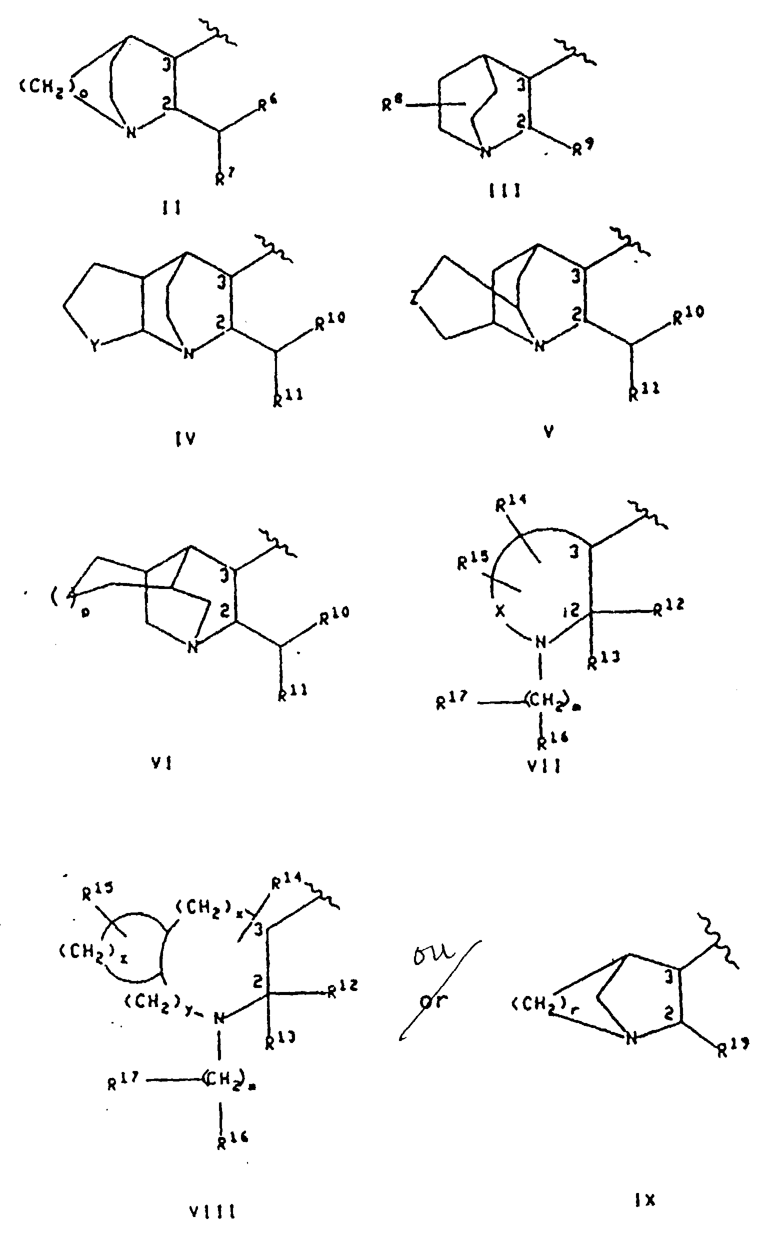
où R⁶ et R¹⁰ sont choisis indépendamment entre furyle, thiényle, pyridyle, indolyle, biphényle et phényle, le noyau phényle en question pouvant facultativement être substitué avec un ou deux substituants choisis indépendamment entre halogéno, alkyle en C₁ à C₁₀ facultativement substitué avec un à trois atomes de fluor, alkoxy en C₁ à C₁₀ facultativement substitué avec un à trois atomes de fluor, carboxy, benzyloxycarbonyle et (alkoxy en C₁ à C₃)-carbonyle ;
R⁴ est un groupe alkyle en C₁ à C₆ ou phényle ;
R⁷ est choisi entre un groupe alkyle ramifié en C₃ ou C₄, un groupe alcényle ramifié en C₅ ou C₆, un groupe cycloalkyle en C₅ à C₇ et les radicaux mentionnés dans la définition de R⁶ ;
R⁸ représente l'hydrogène ou un groupe alkyle en C₁ à C₆ ;
R⁹ et R¹⁹ sont choisis indépendamment entre phényle, biphényle, naphtyle, pyridyle, benzhydryle, thiényle et furyle, et R⁹ et R¹⁹ peuvent facultativement être substitués avec un à trois substituants choisis indépendamment entre halogéno, alkyle en C₁ à C₁₀ facultativement substitué avec un à trois atomes de fluor, et alkoxy en C₁ à C₁₀ facultativement substitué avec un à trois atomes de fluor ;
Y est un groupe (CH₂)ₗ dans lequel l est un nombre entier de 1 à 3, ou bien Y est un groupe de formule : 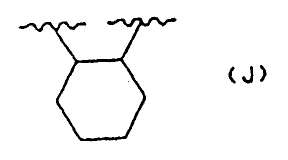
Z est l'oxygène, le soufre, un groupe amino, alkylamino en C₁ à C₃ ou (CH₂)ₙ dans lequel n a la valeur zéro, un ou deux ;
x a la valeur zéro, un ou deux ;
y a la valeur zéro, un ou deux ;
z a la valeur trois, quatre ou cinq ;
o a la valeur deux ou trois ;
p a la valeur zéro ou un ;
r a la valeur un, deux ou trois ;
le noyau contenant (CH₂)_{z} peut contenir zéro à trois doubles liaisons, et l'un des atomes de carbone de (CH₂)_{z} peut facultativement être remplacé par de l'oxygène, du soufre ou de l'azote ;
R¹¹ est un groupe thiényle, biphényle ou phényle facultativement substitué avec un ou deux substituants choisis indépendamment entre halogéno, alkyle en C₁ à C₁₀ facultativement substitué avec un à trois atomes de fluor, et alkoxy en C₁ à C₁₀ facultativement substitué avec un à trois atomes de fluor ;
X est un groupe (CH₂)_{q} dans lequel q est un nombre entier de 1 à 6 et l'une quelconque des liaisons simples carbone-à-carbone dans (CH₂)_{q} peut facultativement être remplacée par une double liaison carbone-à-carbone, et l'un quelconque des atomes de carbone de ce groupe (CH₂)_{q} peut facultativement porter un substituant R¹⁴, et l'un quelconque des atomes de carbone du groupe (CH₂)_{q} peut facultativement porter un substituant R¹⁵ ;
m est un nombre entier de 0 à 8, et l'une quelconque des liaisons simples carbone-à-carbone de (CH₂)ₘ, liaison dont les deux atomes de carbone sont liés l'un à l'autre ou à un autre atome de carbone dans la chaîne (CH₂)ₘ, peut facultativement être remplacée par une double liaison carbone-à-carbone ou par une triple liaison carbone-à-carbone, et l'un quelconque des atomes de carbone du groupe (CH₂)ₘ peut facultativement porter un substituant R¹⁷ ;
R¹² est un radical choisi entre hydrogène, alkyle en C₁ à C₆ linéaire ou ramifié, cycloalkyle en C₃ à C₇ dont l'un des atomes de carbone peut facultativement être remplacé par de l'azote, de l'oxygène ou du soufre ; aryle choisi entre biphényle, phényle, indanyle et naphtyle ; hétéroaryle choisi entre thiényle, furyle, pyridyle, thiazolyle, isothiazolyle, oxazolyle, isoxazolyle, triazolyle, tétrazolyle et quinolyle ; phényl-(alkyle en C₂ à C₆), benzhydryle et benzyle, où le point de fixation sur R¹² est un atome de carbone à moins que R¹² ne soit l'hydrogène et où chacun des groupes aryle et hétéroaryle et des fragments phényle des groupes benzyle, phényl-(alkyle en C₂ à C₆) et benzhydryle en question peut facultativement porter un ou plusieurs substituants choisis indépendamment entre halogéno, nitro, alkyle en C₁ à C₁₀ facultativement substitué avec un à trois atomes de fluor, alkoxy en C₁ à C₁₀ facultativement substitué avec un à trois atomes de fluor, amino, hydroxy(alkyle en C₁ à C₆), (alkoxy en C₁ à C₆)-(alkyle en C₁ à C₆), alkylamino en C₁ à C₆, 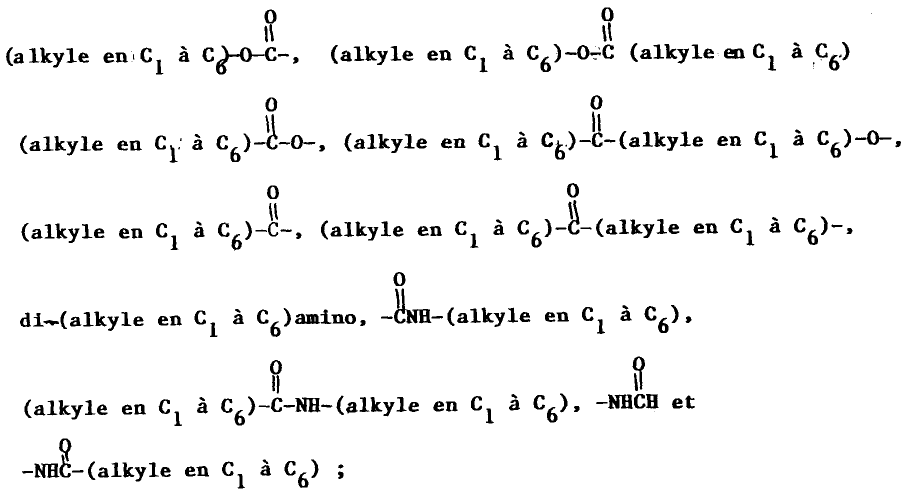 et l'un quelconque des fragments phényle du groupe benzhydryle en question peut facultativement être remplacé par un groupe naphtyle, thiényle, furyle ou pyridyle ;
R¹³ est l'hydrogène, un groupe phényle ou alkyle en C₁ à C₆ ;
ou bien R¹² et R¹³ forment, conjointement avec l'atome de carbone auquel ils sont liés, un noyau carbocyclique saturé ayant 3 à 7 atomes de carbone dans lequel l'un de ces atomes de carbone qui n'est ni le point de fixation du noyau spiro, ni un atome de carbone contigu à ce point peut facultativement être remplacé par de l'oxygène, de l'azote ou du soufre ;
R¹⁴ et R¹⁵ sont tous deux choisis indépendamment entre hydrogène, hydroxy, halogéno, amino, oxo (=O), cyano, hydroxy-(alkyle en C₁ à C₆), (alkoxy en C₁ à C₆)-(alkyle en C₁ à C₆), alkylamino en C₁ à C₆, di(alkyle en C₁ à C₆)amino, alkoxy en C₁ à C₆, 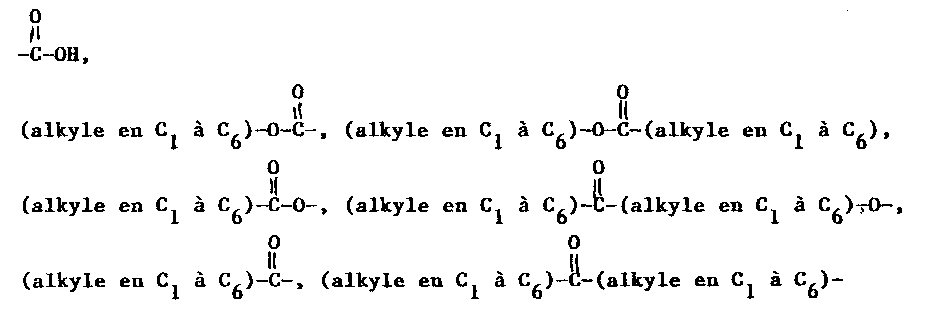 et les radicaux indiqués dans la définition de R¹² ;
R¹⁶ est un groupe 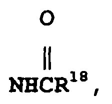 NHCH₂R¹⁸, SO₂R¹⁸, CO₂H ou l'un des radicaux indiqués dans l'une quelconque des définitions de R¹², R¹⁴ et R¹⁵ ;
R¹⁷ est un groupe oximino (=NOH) ou l'un des radicaux indiqués dans l'une quelconque des définitions de R¹², R¹⁴ et R¹⁵ ; et
R¹⁸ est un groupe alkyle en C₁ à C₆, l'hydrogène, un groupe phényle ou phényl-(alkyle en C₁ à C₆) ; sous réserve que (a) lorsque m est égal à 0, l'un de R¹⁶ et R¹⁷ soit absent et l'autre soit l'hydrogène, (b) lorsque R³ est un groupe de formule VIII, R¹⁴ et R¹⁵ ne puissent pas être liés au même atome de carbone, (c) lorsque R¹⁴ et R¹⁵ sont liés au même atome de carbone, chacun de R¹⁴ et R¹⁵ soit choisi indépendamment entre hydrogène, fluoro, alkyle en C₁ à C₆, hydroxy(alkyle en C₁ à C₆) et (alkoxy en C₁ à C₆)-(alkyle en C₁ à C₆), ou bien R¹⁴ et R¹⁵, conjointement avec l'atome de carbone auquel ils sont liés, représentent un noyau carbocyclique saturé en C₃ à C₆ qui forme un composé spiro avec le noyau azoté auquel ils sont liés ; (d) R¹² et R¹⁴ ne puissent pas être tous deux de l'hydrogène ; et (e) lorsque R¹⁴ ou R¹⁵ est lié à un atome de carbone de X ou de (CH₂)_{y} qui est adjacent à l'azote du noyau, R¹⁴ ou respectivement R¹⁵ doive être un substituant dont le point de fixation est un atome de carbone ;
ou un sel pharmaceutiquement acceptable de ce composé.

2. Composé suivant la revendication 1, dans lequel R¹ est choisi entre thiazolyle, azétidinyle, pyrrolyle, pyrazolyle, 1,2,3-triazolyle, 1,2,4-triazolyle, isothiazolyle, imidazolyle, isoxazolyle, oxazolyle, pyridyle, pyrimidinyle, pyrazolyle, thiadiazolyle et thiophényle.

3. Composé suivant la revendication 1 ou la revendication 2, dans lequel les substituants en positions "2" et "3" du noyau azoté de R³ sont en configuration cis.

4. Composé suivant l'une quelconque des revendications 1 à 3, dans lequel R³ est un groupe de formule III, VII ou IX ; R² est l'hydrogène ; A est un groupe phényle ou indolinyle ; W est un groupe alkoxy en C₁ à C₃ facultativement substitué avec un à cinq atomes de fluor ; et R¹ est un groupe thiazolyle, imidazolyle, thiadiazolyle, pyrrolyle, pyridyle, pyrimidinyle, pyrazolyle, thiophényle ou oxazolyle, R¹ pouvant facultativement porter un ou deux substituants alkyle en C₁ à C₃.

5. Composé suivant l'une quelconque des revendications 1 à 4, dans lequel : (a) R³ est un groupe de formule III et R⁹ est le groupe benzhydryle ; (b) R³ est un groupe de formule VII, R¹² est le groupe phényle, chacun de R¹³, R¹⁴, R¹⁵ et R¹⁶ est l'hydrogène, m est égal à zéro et X est le groupe -(CH₂)₃- ; ou bien (c) R³ est un groupe de formule IX, r est égal à deux et R¹⁹ est le groupe benzhydryle.

6. Composé suivant la revendication 1 ou la revendication 2, dans lequel R³ est un groupe de formule III dont les substituants en positions "2" et "3" du noyau azoté sont en configuration cis, R⁹ est le groupe benzhydryle et A est le groupe phényle.

7. Composé suivant la revendication 1, dans lequel R³ est un groupe de formule VII dont R¹² et le substituant en position "3" du noyau azoté sont en configuration cis, A est le groupe phényle, R¹² est le groupe phényle, chacun de R², R¹³, R¹⁴, R¹⁵ et R¹⁶ représente l'hydrogène, m est égal à zéro, W est un groupe méthoxy ou isopropoxy, X est le groupe -(CH₂)₃- et R¹ est un groupe thiazolyle, imidazolyle, pyrrolyle, oxazolyle, pyridyle, pyrimidinyle, pyrazolyle, thiophényle ou thiadiazolyle.

8. Composé suivant la revendication 1, dans lequel R³ est un groupe de formule VII, chacun de R¹³, R¹⁴, R¹⁵ et R¹⁶ est l'hydrogène, m est égal à zéro, X est le groupe -(CH₂)₃-, A est le groupe phényle, W est le groupe méthoxy et R¹ est choisi entre thiazolyle, imidazolyle, thiadiazolyle, pyrrolyle, pyridyle, pyrimidinyle, pyrazolyle, thiophényle et oxazolyle.

9. Composé de formule : 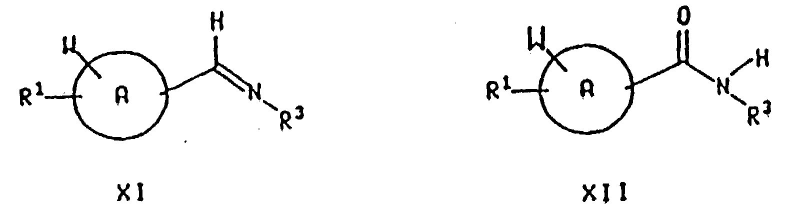 dans laquelle W, A, R¹ et R³ sont tels que définis dans la revendication 1.

10. Composé suivant la revendication 1, choisi dans le groupe consistant en :
(2S,3S)-[5-(2,5-diméthyl-pyrrole-1-yl)-2-méthoxybenzyl]-(2-phénylpipéridine-3-yl)amine ;
(2S,3S)-3-[2-méthoxy-5-(2-thiazolyl)benzyl]amino-2-phénylpipéridine ;
(2S,3S)-3-[5-imidazolyl-2-méthoxybenzyl]amino-2-phénylpipéridine ;
(2S,3S)-3-[2-méthoxy-5-(2-oxopyrrolidinyl)benzyl]amino-2-phénylpipéridine ;
(2S,3S)-3-[2-méthoxy-5-(4-méthyl-2-thiazolylbenzyl]amino-2-phénylpipéridine ;
(2S,3S)-3-[2-méthoxy-5-(1,2,3-thiadiazole-4-yl)-benzyl]amino-2-phénylpipéridine ;
(2S,3S)-3-[2-méthoxy-5-(5-oxazolyl)benzyl)amino-2-phénylpipéridine ;
(2S,3S)-(2-méthoxy-5-pyridine-2-ylbenzyl)-(2-phénylpipéridine-3-yl)amine ;
(2S,3S)-(2-méthoxy-5-pyrimidine-2-ylbenzyl)-(2-phénylpipéridine-3-yl)amine ;
(2S,3S)-(2-méthoxy-5-pyridine-3-ylbenzyl)-(2-phénylpipéridine-3-yl)amine ;
(2S,3S)-[2-méthoxy-5-(6-méthylpyridine-2-yl)benzyl]-(2-phénylpipéridine-3-yl)amine ;
(2S,3S)-[5-(3,5-diméthylpyrazole-1-yl)-2-méthoxybenzyl]-(2-phénylpipéridine-3-yl)amine ;
(2S,3S)-[2-méthoxy-5-(3,4,5-triméthylpyrazole-1-yl)-benzyl]-(2-phénylpipéridine-3-yl)amine ;
(2S,3S)-[2-isopropoxy-5-(3,4,5-triméthylpyrazole-1-yl)benzyl]-(2-phénylpipéridine-3-yl)amine ;
(2S,3S)-[5-(3,5-diisopropylpyrazole-1-yl)-2-méthoxybenzyl]-(2-phénylpipéridine-3-yl)amine ; et
(2S,3S)-[5-(3,5-diméthylthiophène-2-yl)-2-méthoxybenzyl]-(2-phénylpipéridine-3-yl)amine.

11. Composition pharmaceutique destinée au traitement curatif ou préventif d'une pathologie choisie dans le groupe consistant en troubles inflammatoires, anxiété, incontinence urinaire, troubles gastro-intestinaux, dépression ou troubles pathologiques de l'humeur, psychose, douleur, allergies, affections obstructives chroniques des voies respiratoires, troubles d'hypersensibilité, affections angiospastiques, formation de tissu fibreux et maladies du collagène, algodystrophie sympathique réflexe, toxicomanie, troubles somatiques en rapport avec le stress, neuropathie périphérique, névralgie, troubles neuropathologiques, troubles en rapport avec la facilitation immunologique ou l'immunosuppression et affections rhumatismales chez un mammifère, comprenant une quantité d'un composé suivant la revendication 1 efficace pour prévenir ou traiter la pathologie en question, et un support acceptable du point de vue pharmaceutique.

12. Composition pharmaceutique destinée à antagoniser les effets de la substance P chez un mammifère, comprenant une quantité efficace pour antagoniser la substance P d'un composé suivant la revendication 1 et un support acceptable du point de vue pharmaceutique.

13. Composition pharmaceutique destinée au traitement curatif ou préventif d'une pathologie chez un mammifère, dont le traitement curatif ou préventif est réalisé ou facilité par une réduction de la neurotransmission sous la médiation de la substance P, comprenant une quantité d'un composé suivant la revendication 1 efficace pour antagoniser l'effet de la substance P au niveau d'un site récepteur, et un support acceptable du point de vue pharmaceutique.

14. Composition pharmaceutique destinée au traitement curatif ou préventif d'une pathologie chez un mammifère, dont le traitement ou la prévention est réalisé ou facilité par une réduction de la neurotransmission sous la médiation de la substance P, comprenant une quantité d'un composé suivant la revendication 1 ou d'un sel pharmaceutiquement acceptable de ce composé, efficace pour le traitement curatif ou préventif de cette pathologie, et un support acceptable du point de vue pharmaceutique.
